# EUROPEAN PATENT APPLICATION

(11) **EP 4 151 171 A1**
(43) Date of publication of application: **22.03.2023**
(21) Application number: 22195810.1
(22) Date of filing: 15.09.2022
(51) Int. Cl.: A61B 34/10, A61B 90/14, A61B 34/00, A61B 34/20, A61B 90/13, A61B 90/00, A61B 17/00

(54) **ROBOT SURGICAL PLATFORM FOR CRANIAL SURGERY**

(30) Priority: 15.09.2021 US 202117475408
(71) Applicant: Globus Medical, Inc., Audubon, PA 19403 (US)
(72) Inventor: MANTZAVINOS, Spiros, NASHUA, 03064 (US); WIRZ, Raul, MEDFORD, 02155 (US); CASCARANO, James, CAMBRIDGE, 02140 (US); JOHNSON, Norbert, NORTH ANDOVER, 01845 (US); CAMERON, Hayden, PHILADELPHIA, 19129 (US); CRAWFORD, Neil R., CHANDLER, 85224 (US)
(74) Representative: Morabito, Sara

(57) **Abstract**

A cranial surgery planning system including at least one network interface connectable to obtain radiological patient images generated by a radiological image scanner, a display device, at least one processor, and at least one memory storing program code that is executed by the at least one processor. The operations include obtaining (4700) through the at least one network interface a first radiological patient image of a cranial structure of a patient along a first plane and obtaining a second radiological patient image of the cranial structure of the patient along a second plane that is angularly offset to the first plane. The operations also include merging (4702) the first and second radiological patient images to an image coordinate system. The operations also include obtaining (4704) a surgical trajectory plan defining an entry point on the patient's skull and a target point in the patient's brain captured in the merged first and second radiological patient images.

## Description

### TECHNICAL FIELD

The present disclosure relates to medical devices, and more particularly, robotic surgical systems and related methods and devices.

### BACKGROUND

Various medical procedures for cranial surgery require the precise localization of a three-dimensional position of a surgical instrument within the body of a patient in order to effect optimized treatment. Robot surgical platforms are being introduced that can assist surgeons with positioning surgical tools and performing surgical procedures within a patient body. A robot surgical platform can include a robot that is coupled to an end-effector element, and where the robot is configured to control movement and positioning of the end-effector relative to the body. The end-effector may be a surgical tool guide tube, such as a drill guide tube, or may be the surgical tool itself.

There is a need for a robot surgical platform that provides accurate localization of a three-dimensional position of a surgical tool relative to the body in order to effect optimized treatment. Improved localization accuracy can minimize human and robotic error while allowing fast and efficient surgical process. The ability to perform operations on a patient with a robot surgical platform and computer software can enhance the overall surgical procedure and the results achieved for the patient.

### SUMMARY

Some embodiments of the present disclosure are directed to a cranial surgery planning system that includes at least one network interface connectable to obtain radiological patient images generated by a radiological image scanner, a display device, at least one processor, and at least one memory storing program code that is executed by the at least one processor. The processor is configured to perform operations that include obtaining through the at least one network interface a first radiological patient image of cranial structure of a patient along a first plane and obtain a second radiological patient image of the cranial structure of the patient along a second plane that is angularly offset to the first plane. Operations also include merging the first and second radiological patient images to an image coordinate system. Operations also include obtaining a surgical trajectory plan defining an entry point on the patient's skull and a target point in the patient's brain captured in the merged first and second radiological patient images.

The processor is provided inside the robot base. The scanner, not visible in the Figures, could be an imaging system suitable for obtaining images.

Some further embodiments of the present disclosure are directed to the operations of merging the first and second radiological patient images to the image coordinate system, which can include generating a three dimensional graphical representation in the image coordinate system of cranial structure captured in the first and second radiological patient images.

Some further embodiments of the present disclosure are directed to the operations for obtaining the surgical trajectory plan, which can include receiving a user designation of the entry point on the patient's skull and the target point in the patient's brain defined relative to the image coordinate system. The user designation of the entry point and the target point is then stored in the surgical trajectory plan.

Some further embodiments of the present disclosure are directed to operations to obtain the surgical trajectory plan, which can include receiving a user designation of the target point in the patient's brain defined relative to the image coordinate system. The operations include generating a set of preset trajectories based on the target point and a knowledgebase of cranial surgical procedures, and receiving a user selection of one of the preset trajectories. The operations include determining the entry point on the patient's skull based on the selected one of the preset trajectories, and generating the surgical trajectory plan based on the user designation of the target point and the determined the entry point.

Still other cranial surgery planning systems, methods, and computer program products according to embodiments of the inventive subject matter will be or become apparent to one with skill in the art upon review of the following drawings and detailed description. It is intended that all such cranial surgery planning systems, methods, and computer program products be included within this description, be within the scope of the present inventive subject matter, and be protected by the accompanying claims. Moreover, it is intended that all embodiments disclosed herein can be implemented separately or combined in any way and/or combination.

### DESCRIPTION OF THE DRAWINGS

The accompanying drawings, which are included to provide a further understanding of the disclosure and are incorporated in a constitute a part of this application, illustrate certain nonlimiting embodiments of inventive concepts. In the drawings:
Figure 1 illustrates a camera stand;
Figure 2 illustrates a patient stabilization stand;
Figure 3 illustrates the robotic base station;
Figure 4 illustrates a control panel located at the rear of the Robotic Base Station;
Figure 5 illustrates a connector panel located at the rear of the Robotic Base Station;
Figure 6 illustrates 5 axes of motion of the robotic arm;
Figure 7 illustrates a bracelet located at the distal end of the lower arm;
Figure 8 illustrates the parts of the camera stand;
Figure 9 illustrates a 3-pin fixation adapter;
Figure 10 illustrates a Leksell frame base adapter;
Figure 11 illustrates a CRW frame base adapter;
Figure 12 illustrates an Interchangeable Guide End Effector;
Figure 13 illustrates the Interchangeable Guide End Effector motion when moving from one trajectory to the next;
Figure 14 illustrates a verification probe;
Figure 15 illustrates a trajectory probe;
Figure 16 illustrates a navigated biopsy needle;
Figure 17 illustrates an end effector guide tube;
Figure 18 illustrates an electrode drive clamp;
Figure 19 illustrates a 2.5 mm hex driver;
Figure 20 illustrates a cranial drill;
Figure 21 illustrates a cranial drill stop;
Figure 22 illustrates a landmark stylet;
Figure 23 illustrates a cranial adjustable drill guide;
Figure 24 illustrates the front of a Cranial Dynamic Reference Base;
Figure 25 illustrates the back of a Cranial Dynamic Reference Base;
Figure 26 illustrates a Cranial Articulating Arm;
Figure 27 illustrates kinematic mounts located on a Cranial Articulating Arm;
Figure 28 illustrates two versions of a Frame Reference Array;
Figure 29 illustrates a Intraoperative CT Registration Fixture;
Figure 30 illustrates a Fluoroscopy Registration Fixture;
Figure 31 illustrates navigation camera positioning relative to the O.R. table to have the patient reference in the field of view of the navigation camera;
Figure 32 illustrates pressing of the laser button to align the navigation camera;
Figure 33 illustrates a marker attached to a marker post;
Figure 34 illustrates an example of merging images in the cranial application;
Figure 35 illustrates the image coordinate system window of the in the cranial application;
Figure 36 illustrates the planning window of the in the cranial application;
Figure 37 illustrates the patient fixation and patient stabilization;
Figure 38 illustrates an example of a ICT Registration Clamp attached to the Cranial Articulating Arm;
Figure 39 illustrates an example of image acquisition and registration in the cranial application;
Figure 40 illustrates an example of image acquisition and registration of a Leksell frame in the cranial application;
Figure 41 illustrates an example of coordinates of a trajectory in the cranial application;
Figure 42 illustrates an example of CRW localizer fiducial detection in the cranial application;
Figure 43 illustrates an example of calculated frame ring and arc coordinates obtained for each of the planned trajectories in the cranial application;
Figure 44 illustrates an example of images in the cranial application after the ICT Registration Fixture and Clamp are removed;
Figure 45 illustrates an example of images in the cranial application used to monitor the surveillance markers of instruments or implants during a procedure;
Figure 46 illustrates an example of implant placement accuracy verification in the cranial application; and
Figures 47-50 illustrates flowcharts of operations that may be performed by a cranial surgery planning system which is configured according to embodiments

### DETAILED DESCRIPTION

The following discussion is presented to enable a person skilled in the art to make and use embodiments of the present disclosure. Various modifications to the illustrated embodiments will be readily apparent to those skilled in the art, and the principles herein can be applied to other embodiments and applications without departing from embodiments of the present disclosure. Thus, the embodiments are not intended to be limited to embodiments shown, but are to be accorded the widest scope consistent with the principles and features disclosed herein. The following detailed description is to be read with reference to the figures, in which like elements in different figures have like reference numerals. The figures, which are not necessarily to scale, depict selected embodiments and are not intended to limit the scope of the embodiments. Skilled artisans will recognize the examples provided herein have many useful alternatives and fall within the scope of the embodiments.

### SYSTEM OVERVIEW

A cranial surgery planning system 100, which may also be referred to as a ExcelsiusGPS Cranial Module 100, includes five main components: Robotic Base Station 3, Camera Stand 1, Patient Stabilization Stand 2, Instruments 4, and the Cranial Software Module 5, in accordance with some embodiments of the present disclosure.

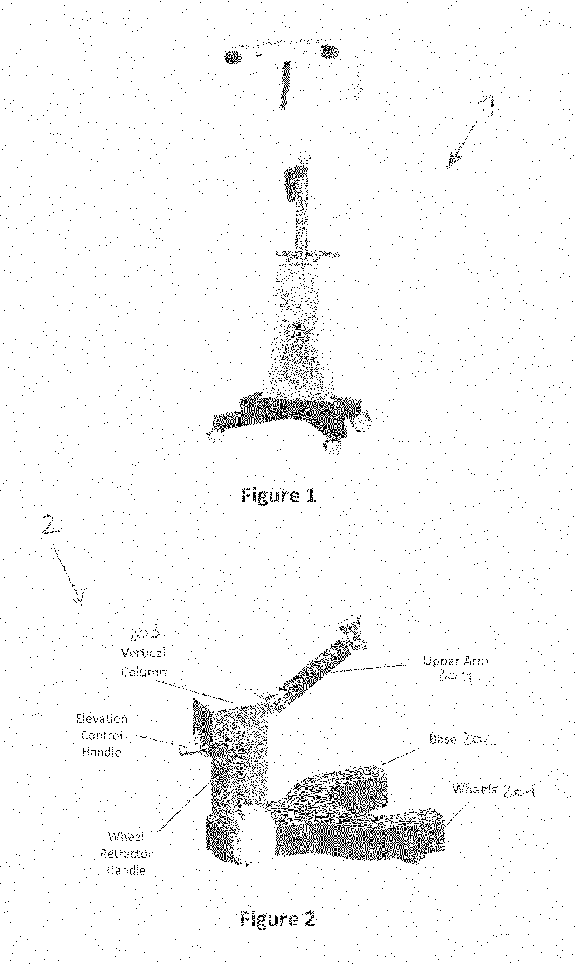

Figure 1 illustrates a camera stand 1.

Figure 2 illustrates a patient stabilization stand 2.

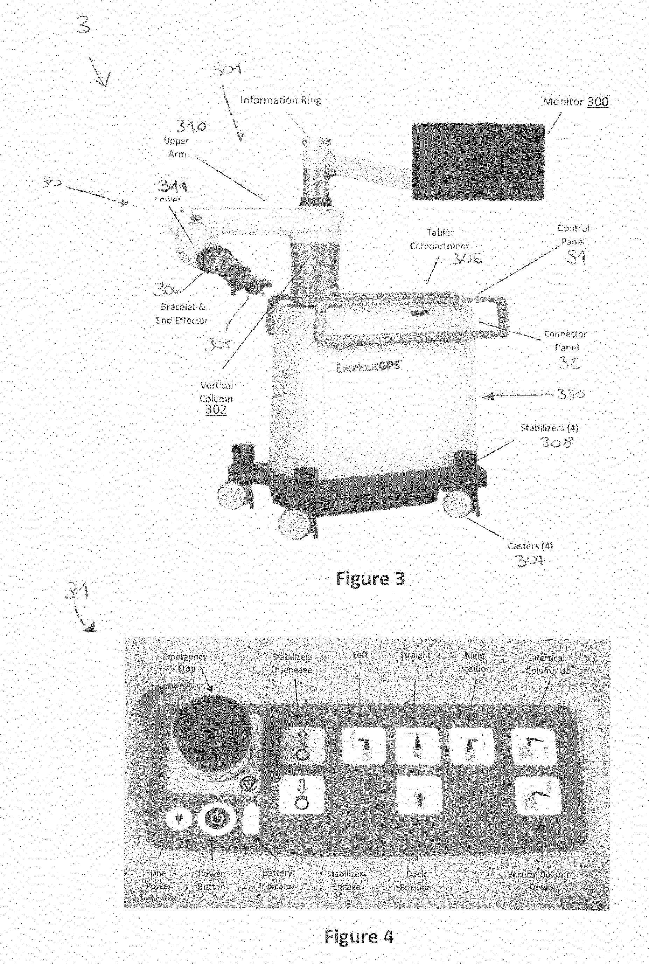

Figure 3 illustrates the robotic base station 3. The robotic base station 3is the main control center for the cranial surgery planning system 100 (e.g., ExcelsiusGPS). The monitor 300 allows the surgeon to plan the surgery and visualize anatomical structures, instruments, and implants in real time. The monitor 300 can be a high resolution, flat panel touch screen liquid crystal display (LCD) located on the vertical column 302. The monitor 300 can be adjusted to the desired location with two hands. An external mouse is available for optional use with the monitor 300. The mouse is not intended for use within the sterile field.

An optional wireless tablet is available for use as a second touchscreen monitor for operative planning and software control. The main monitor 300 remains active at all times during use. The user can lockout tablet use if desired. The tablet compartment 306 is used to store the tablet. The tablet is not intended for use within the sterile field.

Figure 4 illustrates a control panel 31 located at the rear of the Robotic Base Station 3. This control panel 31 is used to display and control system power and general positioning functions.

Control panel functions are illustrated in Figure 4 and reported in the table below.

| Button | Function | To Use |
|---|---|---|
| Emergency Stop | Removes power from motors and applies brake | Press down to activate. Tc deactivate and re-power, twist knob counterclockwise. |
| Line Power Indicator | Illuminates when system is plugged into AC power outlet | Press to turn ON/OFF |
| Power Button | Powers the Robotic Base Station ON/OFF. Illuminated when ON. | Press to turn ON/OFF |
| Battery Indicator | Indicates level and state of charge All bars are illuminated when fully charged | |
| | When operating on battery, number of illuminated bars indicates percent of charge | |
| | Bars progressively illuminate when charging | |
| Stabilizers Disengage | Illuminates when system is free to move | Press to disengage the stabilizers to allow movement of the system |
| Stabilizers Engage | Illuminates when system is secured to floor | Press to engage the stabilizers, to lock the system in place |
| Left Position | Moves upper arm forward and lower arm at a 90° angle to the left | Press and hold button. Operator may release button prior to final position and arm will stop in current position. |
| Right Position | Moves upper arm forward and lower arm at a 90° angle to the right. | |
| Straight Position | Moves upper and lower arm forward | Stop in current position |
| Dock Position | Moves upper and lower arm to rest over the cabinet | |
| Vertical Column Up | Moves vertical column up | Press and hold button. Operator should release button once the desired height is reached. |
| Vertical Column Down | Moves vertical column down | |

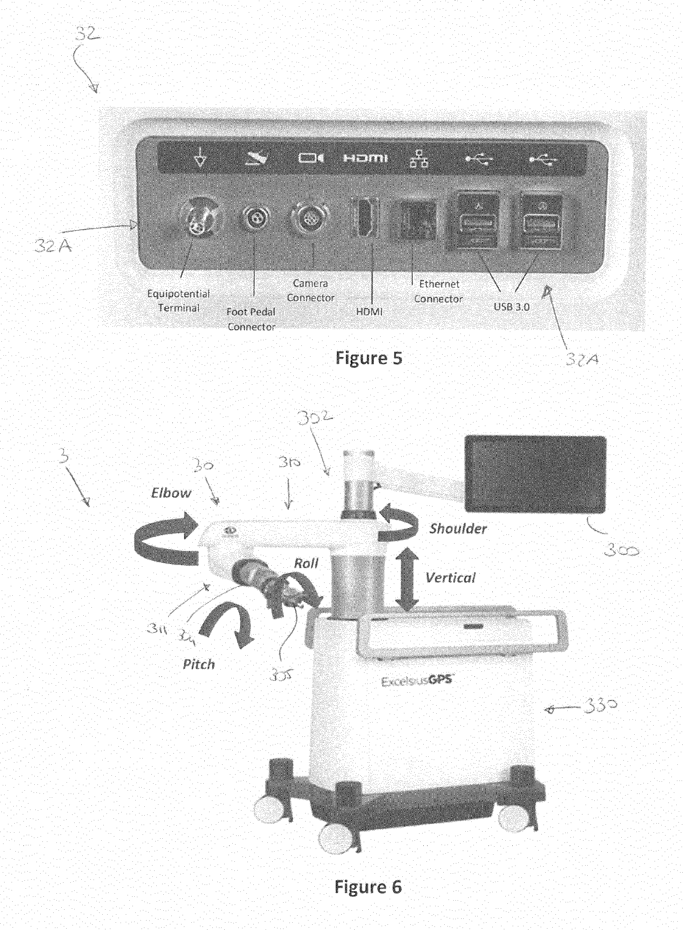

Figure 5 illustrates a connector panel 32 located at the rear of the Robotic Base Station 3. This connector panel 32 contains external connection ports 32A for various devices.

Connector panel 32 functions are illustrated in Figure 5 and reported in the table below.

| Item | Function |
|---|---|
| Equipotential Terminal | Used to connect to other auxiliary equipment; used by service personnel |
| Foot Pedal Connector | Connects to the foot pedal cable |
| Camera Connector | Connects to the camera stand cable |
| HDMI Connector | Connects to an external monitor |
| Ethernet Connector | Connects to a network or intra-operative imaging system for image transfer |
| USB Port 3.0 | Connects to a USB device for image transfer |
| | Connects to C-Arm via video capture supplied with the Fluoroscopy Registration Fixture |

The robotic base station 3 system includes four casters 307 with integrated stabilizers 308. The stabilizers 308 are used to immobilize the robotic base station 3 to ensure that it does not move during use.

The robotic base station 3 further comprises a robotic arm 30, which includes an upper arm 310 and lower arm 311, is attached to the vertical column 302 of the cranial surgery planning system (e.g., ExcelsiusGPS Robotic Base Station 3). This configuration allows for a wide range of motion.

The robotic arm is movable in relation to the vertical column 302. The cranial surgery planning system (e.g., ExcelsiusGPS) may employ an advanced drive control system along with high performance servo drives to accurately position and control the 5-axis robotic arm 301 in an operating room environment. Figure 6 illustrates the 5 axes of motion of the robotic arm 301.

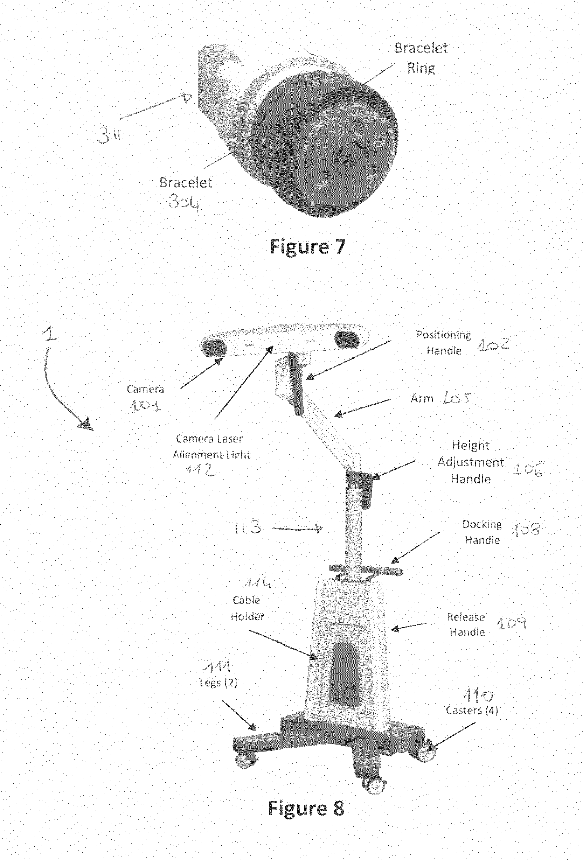

The lower arm comprises a distal end at which a bracelet 304 is attached. Figure 7 illustrates a bracelet 304 located at the distal end of the lower arm 311. It is a load-sensing component that allows user guided positioning of the robotic arm 301.

To initiate motion, squeeze the ring of the bracelet 304 with the thumb and forefinger on opposite sides. While squeezed, apply light force toward the desired direction of motion. The robotic arm 311 will move in the desired direction. The robotic arm 301 moves manually in any direction or along a trajectory, without exceeding the desired depth, if a plan is active.

The camera stand 1 comprises a camera 101 and it is mobile and adjustable to position the camera 101 to view the operating field and optical markers. Figure 8 illustrates the parts of the camera stand 1.

The following table describes the camera stand functions of the parts illustrated in Figure 8 and their reference number.

| Ref. number | Item | Function |
|---|---|---|
| 101 | Camera | Used to detect the reflective markers and is attached to the top of the camera stand. For more information, please refer to the NDI Passive Polaris Spectra User Guide. |
| 102 | Positioning Handle | Used to adjust the camera position to ensure the surgical field is in view. |
| 103 | Handle Tilt Button | Used to adjust the angle of the positioning handle with respect to the camera in the field of view. |
| 104 | Laser Button | Turns the camera laser alignment light on and off. The laser light is used for assistance in aligning the camera in the field of view. |
| 105 | Arm | Provides a large range of positions for the camera. |
| 106 | Height Adjustment Handle | Allows for adjustment of camera height. |
| 107 | Locking Handle | Used to lock camera position. |
| 108 | Docking Handle | Used to collapse the legs for docking the camera stand into the Robotic Base Station. |
| 109 | Release Handle | Releases the camera from the Robotic Base Station. |
| 110 | Casters | The camera stand contains four casters. The rear casters are lockable to prevent the camera stand from moving. |
| 111 | Legs | The camera stand legs swing inward for docking and outward when deployed. |
| 112 | Cable Holder | Provides storage for the camera stand cable. |

Referring to Figure 2, the Patient Stabilization Stand 2 is a portable stand that may be rigidly fixed to the floor, such as at the head of the operating room table. It includes wheels 201, base 202, vertical column 203, and upper arm 204. The vertical column 203 and upper arm 204 are adjustable to allow for a wide range of surgical configurations

The patient is positioned on the operating room (O.R.) table and a patient fixation device, such as a stereotactic frame or Mayfield system, is attached to the patient. The stand 2 is positioned and the upper arm 204 is coupled to the patient fixation device. The stand features a mechanism to retract the wheels 201 and secure the stand 2 on the floor. This prevents unintended motion of the patient. The design allows for access by an intra-operative imaging system such as a fluoroscopy C-arm or CT device.

The Patient Stabilization Stand 2 includes adapters that attach to the upper arm 204. These adapters couple the stand 2 directly to specific patient fixation devices. The Patient Stabilization stand 2 can be coupled to a 3-pin fixation device (e.g. Mayfield) using a 3-Pin Fixation Adapter. Alternatively, the Leksell Frame Base Adapter or CRW Frame Base Adapter can be attached to the stand if the user desires to position the patient using a stereotactic frame. Each adapter has additional features that can be used to attach Cranial Articulating Arms to facilitate various registration methods

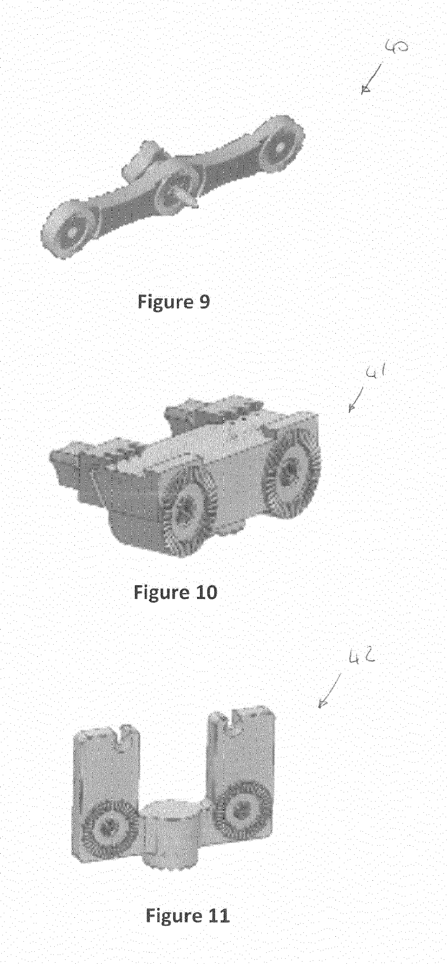

Figure 9 illustrates a 3-pin fixation adapter 40.

Figure 10 illustrates a Leksell frame base adapter 41.

Figure 11 illustrates a CRW frame base adapter 42.

Instruments are now discussed herein.

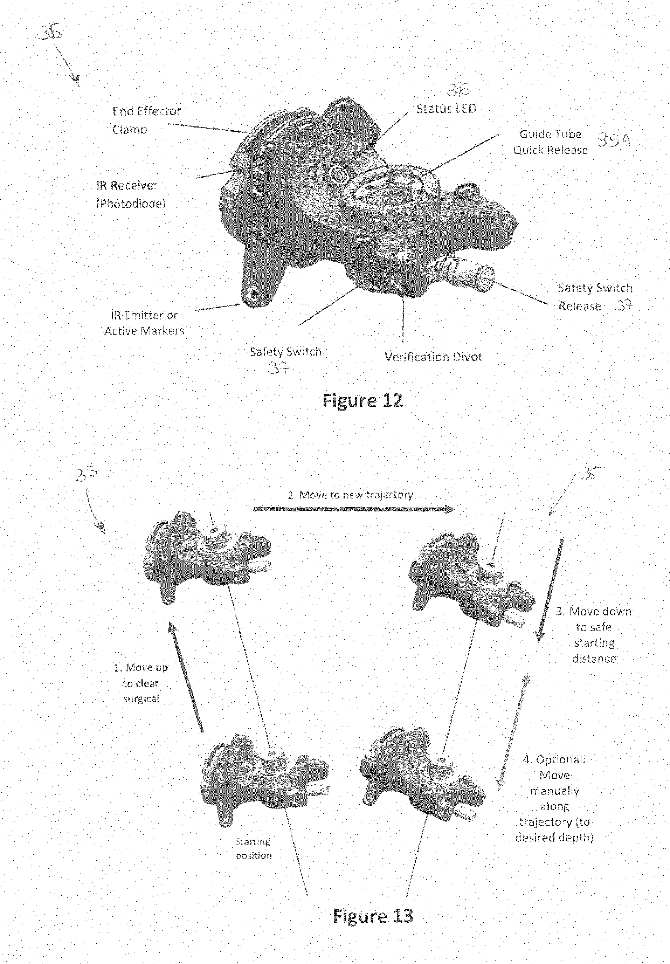

Figure 12 illustrates an Interchangeable Guide End Effector 35. The Interchangeable Guide End Effector 35 is the interface between the robotic arm 30 and the system specific surgical instruments. It allows for a rigid connection through the sterile drape to provide precise positioning of instruments placed within its guide tube. The end effector 305 is provided as a separate component and is sterilized by the user prior to use. Always use care when handling or transporting the end effector as it contains sensitive electronics.

The Interchangeable Guide End Effector 33 is powered wirelessly from the robotic arm 30. This power is used to drive the active markers that are used by the camera to identify the location and orientation of the end effector. The green status LED 36 illuminates when the end effector 305 is powered and is capable of motion. The LED turns off when arm motion is disabled.

The Interchangeable Guide End Effector 35 may be attached to a variety of guide tubes. When a guide tube is attached, various instruments may be inserted through the guide tube along the desired trajectory, including electrode drive clamps, drills with or without drill stops, drill guide, and biopsy needles.

The Interchangeable Guide End Effector 35 includes a Safety Switch. The Safety Switch has two positions: 'Open' and 'Closed'. When 'Open', robotic arm motion is disabled; when 'Closed', robotic arm motion is enabled. For safety, instruments can only be inserted through the end effector when the switch is in the 'Open' position. The switch cannot be 'Closed' until the instrument is removed from the end effector. This will ensure that robotic arm motion does not occur while an instrument is inserted through the End Effector.

To transition from 'Open' to 'Closed', the Safety Switch Release 37 is slid from left to right. To transition from 'Closed' to 'Open', pull out on the Safety Switch Release 37 then the Safety Switch Release 37 is slid from right to left.

An Electro Surgical Unit (ESU) may be used during the procedure adhering to the guidelines below:
1. Maximum peak voltage in Cut mode is 900 Vpk.
2. Maximum peak voltage in Coag mode is 4500 Vpk.
3. The energized ESU probe should not come into contact with the metal end effector itself, contact should only be made to the stylet or curette.
4. If a stylet is used, it must be properly positioned in the electrode drive feeder. The electrode drive feeder must be properly mounted on the end effector using the Electrode Drive Clamp.
5. If a curette is used, it must be inserted through the End Effector Guide Tube. The End Effector Guide Tube is electrically isolated and must be properly mounted in the end effector quick release mechanism.

The cranial surgery planning system (e.g., ExcelsiusGPS) robotic arm 30 positions the Interchangeable Guide End Effector 35 to guide instruments at the desired trajectory. The surgeon manually inserts instruments and implants through the end effector 305 while aligned in the desired trajectory for accurate placement.

Motion of the robotic arm is only allowed with continuous pressing of the bracelet or foot pedal. The arm is manually moved by the user in Wrist mode, or is automatically moved to the selected trajectory in Trajectory mode.

In Wrist mode, the arm may be moved manually to any position within reach of the arm.

In Trajectory mode, the arm is automatically moved from the current position to the next trajectory when ready, or may be moved manually along a selected trajectory.

When moving from one trajectory to the next, the arm moves outwards along the current trajectory to a safe distance (200mm) from the surgical site before moving to the new trajectory and downward along the current trajectory to the anatomy

Automatic motion of the arm occurs when moving the end effector from the current position (either initially or at a current trajectory) to a new trajectory. Once the end effector has moved to a new trajectory, it can only be moved up and down the path of the trajectory, to the desired depth.

Figure 13 illustrates the Interchangeable Guide End Effector 35 motion when moving from one trajectory to the next. Trajectories 1, 2, and 3 are automatic movements. Trajectory 4 is manual and optional.

Automatic motion of the robotic arm may be stopped by the user, stopped by the system, or prevented. To stop motion at any time, the Emergency Stop button located on the base station is pressed. Motion is stopped if the end effector detects a force greater than 50N (11lbs) in the direction opposite of motion. The end effector detects a force greater than 100N (22 lbs) in any direction. Motion is also stopped in Trajectory mode when the patient reference element, such as a Cranial Dynamic Reference Base (CDRB) or Frame Reference Array (FRA) or the end effector is not in view of the camera. Motion is prevented when the safety switch in the Interchangeable Guide End Effector 35 is open. When a trajectory is selected, motion of the arm is only allowed in trajectory mode.

If the robotic arm 30 is not able to reach a safe starting location due to its current position, an error message is shown that indicates that the trajectory is out of reach. A new trajectory must be used or the base must be repositioned.

For a new trajectory, the selected trajectory and positions the robotic arm 30 using the bracelet 304 are cleared to a clear position. The bracelet 304 provides flexibility for the user to move the arm around an obstacle.

To reposition the base, the stabilizers on the casters are disengaged, the station is moved to the desired location and the stabilizers are reengaged. Registration is unaffected because the patient reference has not moved with respect to the patient.

Prior to use, the robotic arm's ability to be moved using the wrist mode and arm position buttons on the control panel is confirmed.

Navigated surgical instruments may include a navigated biopsy needle and navigated probes, such as a verification probe and a trajectory probe.

The Navigated Probes have optical marker arrays parallel with or perpendicular to the instrument axis. The Navigated Probes are used to indicate anatomical structures.

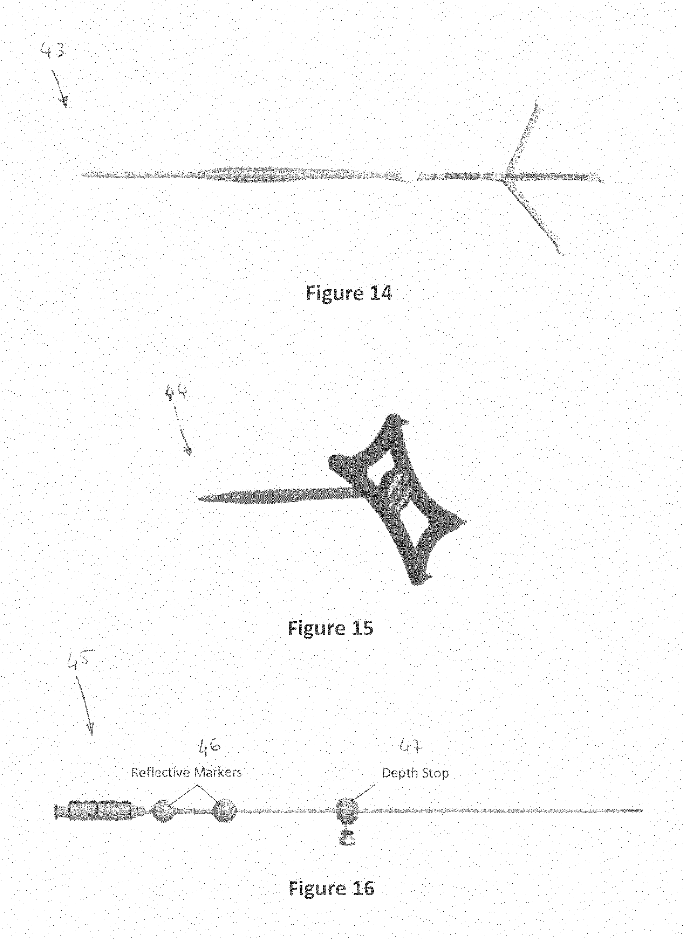

Figure 14 illustrates a verification probe 43.

Figure 15 illustrates a trajectory probe 44.

Figure 16 illustrates a navigated biopsy needle 45. The Navigated Biopsy Needle 45 is a navigated instrument that can be used free hand or through the Interchangeable Guide End Effector 35. When using the needle 45 through the end effector 304, the unique array pattern defined by the reflective markers 46 determine the distal tip location. The array is tracked by the camera and the distal tip location is projected on the monitor. A depth stop 47 may be added to prevent the needle from being inserted beyond the desired depth.

Non-navigated surgical instruments may include an end effector guide tube, a electrode drive clamp, a 2.5 mm hex driver, a cranial drill, a cranial drill stop, a landmark stylet, a cranial adjustable drill guide.

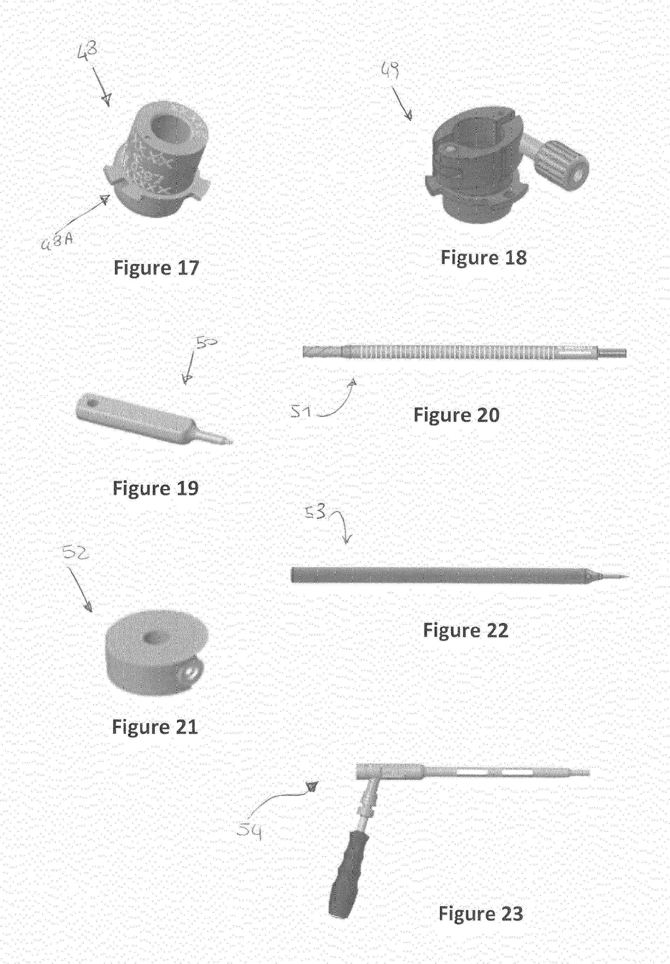

Figure 17 illustrates an end effector guide tube 48.

Figure 18 illustrates an electrode drive clamp 49.

Figure 19 illustrates a 2.5 mm hex driver 50.

Figure 20 illustrates a cranial drill 51.

Figure 21 illustrates a cranial drill stop 52.

Figure 22 illustrates a landmark stylet 53.

Figure 23 illustrates a cranial adjustable drill guide 54.

Non-navigated surgical instruments for use with ExcelsiusGPS include the End Effector Guide Tubes, Electrode Drive Clamp, Cranial Drill Stop, 2.5mm Hex Driver, Cranial Drill, Landmark Stylet, and Cranial Adjustable Drill Guide. Ancillary instruments including electrode drivers, cranial drills, biopsy needles, catheters, and stylets may be inserted through an End Effector Guide Tube inserted into the Interchangeable Guide End Effector. When drills are used, a drill stop is provided to prevent drilling beyond the desired depth

To insert the End Effector Guide Tube 48 into the Interchangeable Guide End Effector 35, the Quick Release 35A is loosened and then the Guide Tube alignment feature 48A is aligned with the alignment pin on the end effector 35. The Quick Release 35A is tightened and the Guide Tube is checked to ensure it is securely connected.

Surveillance marker holders are now discussed.

The Surveillance Marker Holder is a stand-alone instrument that holds one reflective marker. It is used to alert the user if the Cranial Dynamic Reference Base or Frame Reference Array has moved. The Surveillance Marker Holder is attached to patient fixation such as a stereotactic frame base. It is not required for navigation, but is an additional safety measure to monitor movement and confirm that the patient reference (CDRB or FRA) remains fixed in the optical space

Registration instruments, such as CDRBs, Cranial articulating arms, and FRAs, are now discussed.

Registration instruments are used in the patient registration process. Patient registration is a process that creates the correlation between the virtual patient anatomy obtained from patient images, with the physical patient anatomy in the operating room.

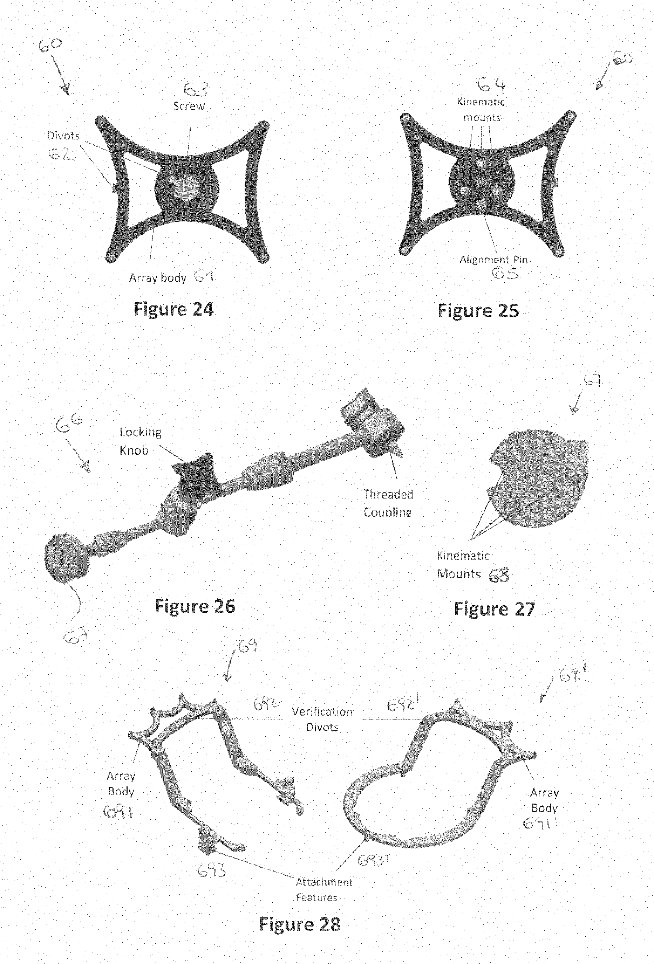

Figure 24 illustrates the front of a Cranial Dynamic Reference Base 60.

Figure 25 illustrates the back of a Cranial Dynamic Reference Base 60.

The Cranial Dynamic Reference Base (CDRB) 60 is a patient reference and is used to establish a fixed reference point in the optical space from which all navigation tracking is referenced. The CDRB 60 is comprised of an array body 61, divots 62, screw 63, kinematic mounts 64, and an alignment pin 65. The CDRB 60 has a unique array pattern (configuration of tracked marker posts) so that it can be identified by the system. The CDRB has divots 62 that are used for instrument verification. The distal end of a navigated instrument is placed in the divot 62 of the CDRB and held in front of the optical camera, to verify the instrument for use.

Figure 26 illustrates a Cranial Articulating Arm 66.

Figure 27 illustrates kinematic mounts 67 located on a Cranial Articulating Arm 66.

The CDRB 60 is mounted to the Cranial Articulating Arm 66 through the CDRB screw 63 and kinematic mounts 64. It has several joints, which are locked or unlocked by tightening or loosening the locking knob to allow for quick adjustments. The kinematic mount 64 and the CRDB's alignment pin 65 allow the CDRB to be removed and replaced intraoperatively without loss of registration.

Figure 28 illustrates two versions of a Frame Reference Array 69, 69A.

The Frame Reference Array (FRA) is an alternative to the CDRB when a stereotactic frame is used during the procedure. Frame Reference Arrays 69, 69' function to establish a fixed reference point in the optical space from which all navigation tracking is referenced. Two FRAs are available; one is compatible with a Leksell stereotactic frame, and one with a CRW stereotactic frame. The FRAs include an array body 691, 691' with verification divots 692, 692' and have attachment features 693, 693' to facilitate attachment to their associated Leksell or CRW frame base.

Registration fixtures, such as an Intraoperative CT Registration Fixture and a Fluoroscopy Registration Fixture, are now discussed.

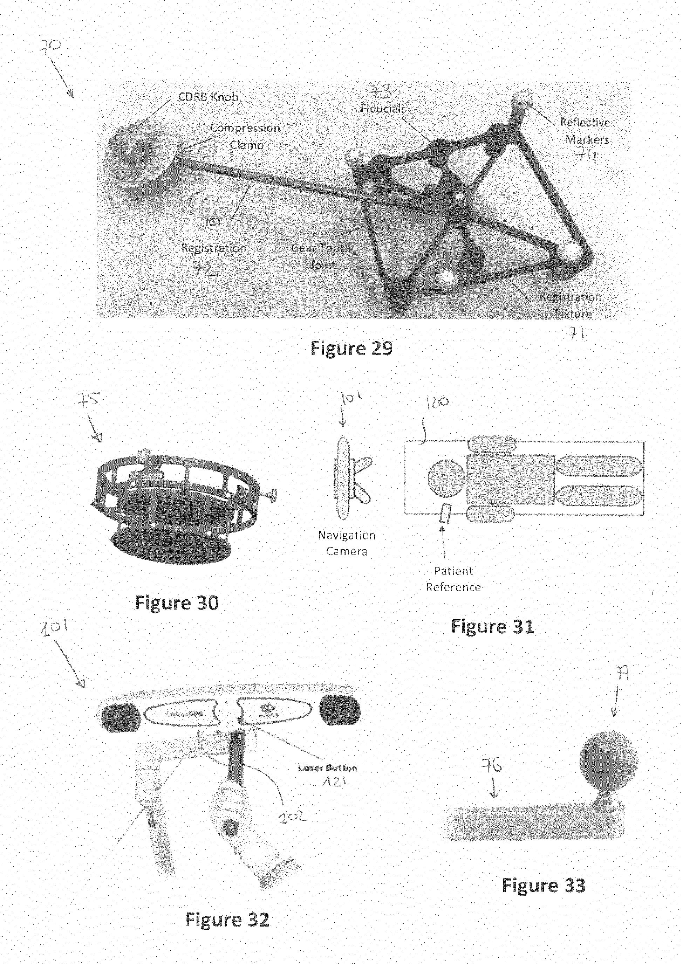

Figure 29 illustrates an Intraoperative CT Registration Fixture 70.

The Intraoperative CT (ICT) Registration Fixture 70, include a registration fixture 71 and ICT registration clamp 72 allows for any intraoperative CT image to be used with the cranial surgery planning system (e.g., ExcelsiusGPS Cranial Module). The ICT Registration Clamp 72 and Registration Fixture 71 are assembled prior to use by matching the starburst gears and snapping the two components together. The Intraoperative Registration Fixture 70 is positioned near the surgical site using the ICT Registration Clamp 72 with the Cranial Articulating Arm. The fiducials 73 are detected automatically in the intraoperative image and are used to register the patient's anatomy during the scan to the patient reference, which is tracked by the camera throughout the procedure. The reflective markers 74 are detected by the camera. Once the registration is transferred to the patient reference, the ICT Registration Fixture 72 is removed to provide access to the surgical site

Figure 30 illustrates a Fluoroscopy Registration Fixture 75.

The Fluoroscopy Registration Fixture 75 allows for any intraoperative fluoroscopic image to be used. The fluoroscopy fixture 75 is attached to the image intensifier of the fluoroscope using the integrated clamps. The fixture should be positioned such that the reflective markers are seen by the camera in all intended fluoroscope positions: Anterior/Posterior (AP), Lateral, et

System setup is now discussed herein.

A camera cord from the cord holder is plugged into the connector panel on the base station.

The camera is moved to the O.R. table and the wheel brakes are engaged by activating the lever located on the wheel. The camera is aligned to view the surgical field.

Figure 31 illustrates navigation camera 101 positioning relative to the O.R. table 120 to have the patient reference in the field of view of the navigation camera.

The laser button 121 located on the positioning handle 102 of the camera is pressed and held to activate the camera's alignment laser and the position is adjusted so the laser points to the center of the surgical field.

Figure 32 illustrates pressing of the laser button 121 to align the laser of the navigation camera 101.

Next, the robotic base station is positioned. The foot is positioned on level ground at a comfortable distance from the operator's feet. The foot pedal is IPX68 rated and is acceptable for use in areas where liquids are likely to be found. Plug the foot pedal cord into the connector panel. The foot pedal allows the arm to move to the active trajectory, similar to the action of the bracelet on the lower arm.

The robotic base station is positioned next to the patient at a comfortable distance from the surgeon. Move the robotic arm, using the bracelet, around the planned trajectories to ensure the arm can reach all locations before engaging the stabilizers.

The stabilizer engage button is pressed on the control panel to lower the stabilizers on the casters. The button is illuminated when the stabilizers are engaged.

Attaching the end effector 305 to the robotic arm 30 is discussed herein. The Interchangeable Guide End Effector 35 connects to the robotic arm 30 through the interface plate over the custom drape. A magnetic assist helps to position and self-align the end effector. The end effector is equipped with a drape-friendly clamp that allows it to be removed and reattached up to three times during a procedure without damaging the drape.

The brackets on the end effector are opened and placed on the interface plate by aligning the V grooves and alignment spheres. The brackets on both sides of the end effector are squeezed and the handle is pressed down to lock into place.

To remove the end effector from the robotic arm, the handle is pull up on to release the spring and side brackets.

Figure 33 illustrates a marker 77 attached to a marker post 76. Attach the disposable reflective markers to each marker posts of each instrument assembly. Ensure that the markers are fully seated on the posts.

Procedures using the Cranial Application use the following workflow.

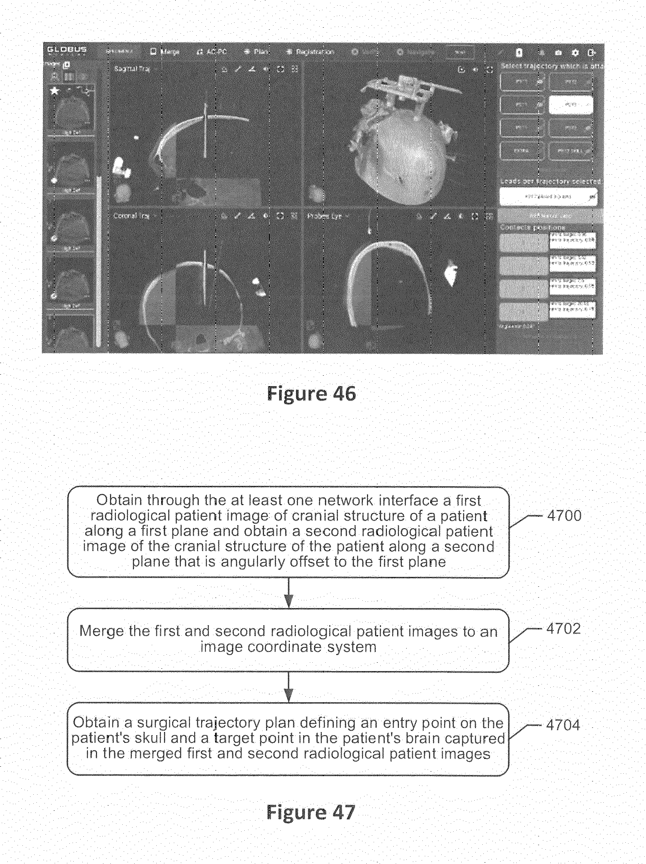

Figure 47 illustrates flowcharts of operations that may be performed by a cranial surgery planning system which is configured according to embodiments. Referring to Figure 47, the cranial surgery planning system includes at least one network interface connectable to obtain radiological patient images generated by a radiological image scanner, a display device 300, at least one processor 330, and at least one memory storing program code that is executed by the at least one processor 330. The network interface is a part of the processor provided in the robot base station 3. The processor is configured to perform operations which include obtaining 4700 through the at least one network interface a first radiological patient image of cranial structure of a patient along a first plane and obtain a second radiological patient image of the cranial structure of the patient along a second plane that is angularly offset to the first plane. Operations also include merging 4702 the first and second radiological patient images to an image coordinate system. Operations also include obtaining 4704 a surgical trajectory plan defining an entry point on the patient's skull and a target point in the patient's brain captured in the merged first and second radiological patient images.

In some embodiments, the first and second radiological patient images are angularly offset in a range between 90° and 30°.

In some embodiments, operations performed by the cranial surgery planning system further include generating a set of preset trajectories based on the target point and a knowledgebase of cranial surgical procedures, by displaying graphical representations of the trajectories as overlays on the first and second radiological patient images. Operations also include receiving the user selection of one of the preset trajectories, by receiving the user selection of one of the displayed graphical representations of the trajectories.

First, images are merged after the images are imported and selected in the Cranial Application. Merging is the process of registering two or more image sets of the same patient anatomy together. This can be any combination of CT or MR image sets. To import and select images, "Add Image" on the "Merge" page is clicked to open the "Image Sets" dialog as seen below. Images can be loaded from a USB drive, hard drive, or network. To view images on a USB drive, insert the USB drive into the USB port on the connector panel. To load an image, select the hard drive or USB drive icon and select the desired patient image. The desired images are selected from the "Local" image list, then a master image is identified. All images are merged to the master image to ensure that exam images and registration images reference the same coordinate system. All images for the case are selected to continue to merge.

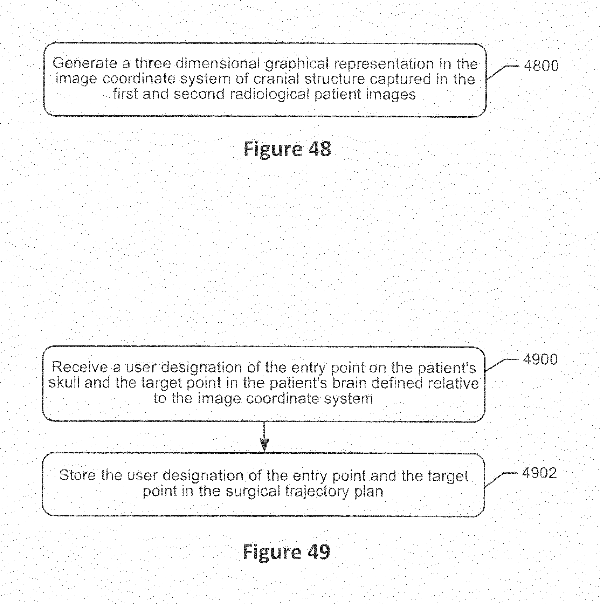

Figure 48 illustrates flowcharts of operations that may be performed by a cranial surgery planning system which is configured according to embodiments. Referring to Figure 48, in some embodiments, the operation of merging 4702 the first and second radiological patient images to the image coordinate system include generating 4800 a three dimensional graphical representation in the image coordinate system of cranial structure captured in the first and second radiological patient images.

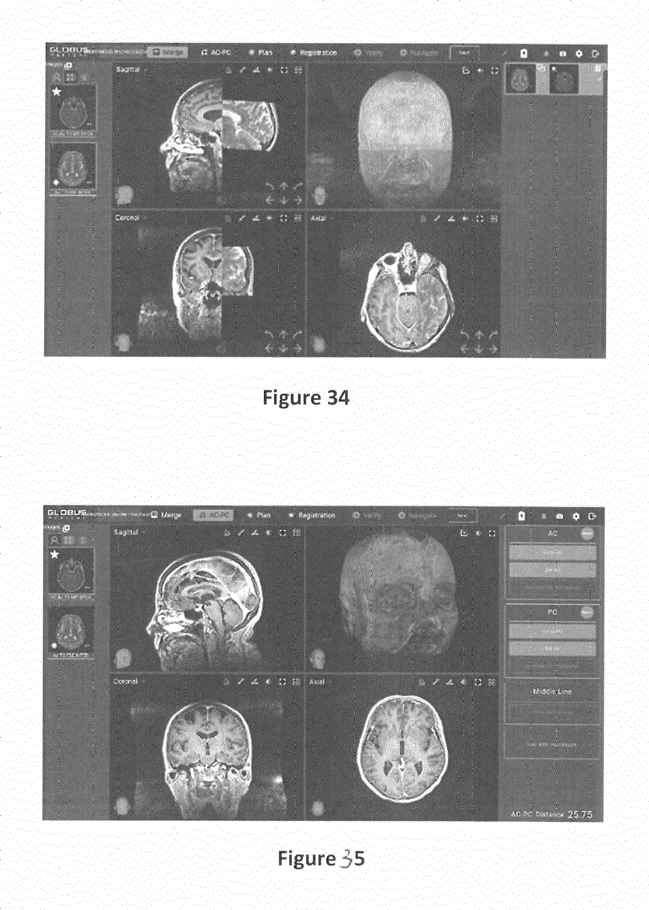

Figure 34 illustrates an example of merging images in the cranial application. To merge the images, the two images are selected to merge, the master image and another image. The software automatically merges the selected image to the master image. The automatic merge using the different view modes can be accessed by clicking one of the image icons. The images are overlaid to determine the quality of the merge, and can be adjusted using the arrows on each image. Once the first image is registered to the master image, the second image can be registered to the first image, rather than to the master. All images should be registered to the master image.

Next, the Coordinate System is defined. Once the images are merged to the master image coordinate system, the user may identify the anterior commissure (AC) and the posterior commissure (PC) anatomical landmarks that define the image coordinate system. The user can bypass this step and select the standard X-Y-Z coordinate system by clicking on "Use scan coordinates."

To set image coordinates, align the red dot shown on each of the four images with the AC or PC anatomical landmark as desired. Once aligned, click "Set AC" or "Set PC". A green dot shows the position on patient images. AC/PC coordinates may be adjusted using the adjustment arrows.

Figure 35 illustrates the image coordinate system window of the in the cranial application.

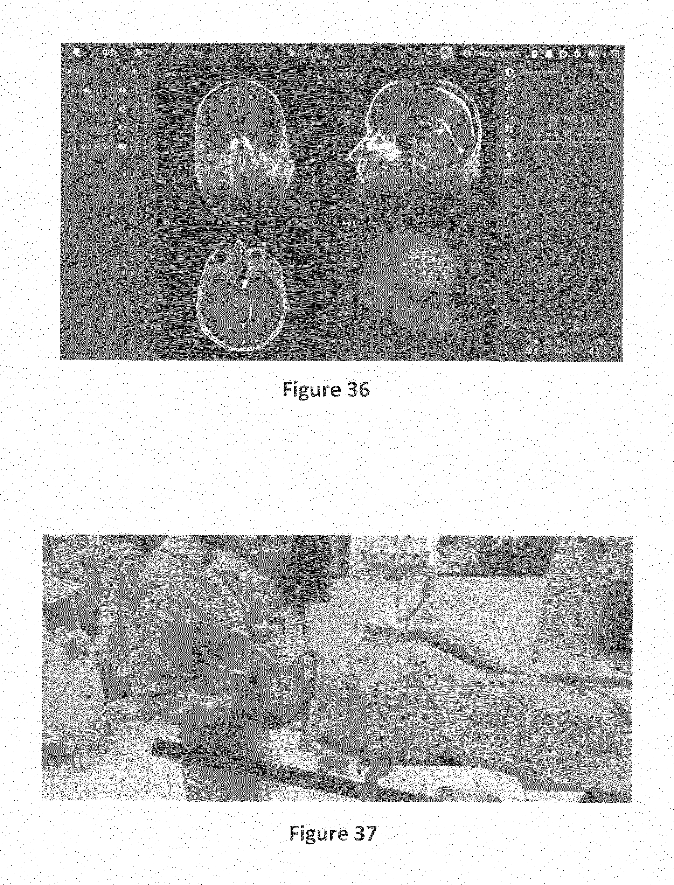

Next, the trajectory is planned. A preset trajectory may be selected, a new trajectory may be planned, or a new preset trajectory may be defined. Figure 36 illustrates the planning window of the in the cranial application.

Figure 49 illustrates flowcharts of operations that may be performed by a cranial surgery planning system which is configured according to embodiments. Referring to Figure 49, operations to obtain 4704 the surgical trajectory plan defining the entry point on the patient's skull and the target point in the patient's brain captured in the merged first and second radiological patient images include receiving 4900 a user designation of the entry point on the patient's skull and the target point in the patient's brain defined relative to the image coordinate system. The operations also include storing 4902 the user designation of the entry point and the target point in the surgical trajectory plan.

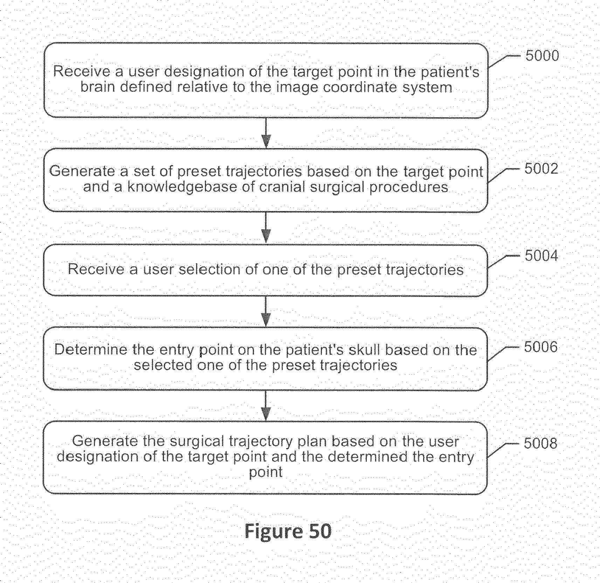

Figure 50 illustrates flowcharts of operations that may be performed by a cranial surgery planning system which is configured according to embodiments. Referring to Figure 50, operations to obtain 4704 the surgical trajectory plan defining the entry point on the patient's skull and the target point in the patient's brain captured in the merged first and second radiological patient images includes receiving 5000 a user designation of the target point in the patient's brain defined relative to the image coordinate system. The operations also include generating 5002 a set of preset trajectories based on the target point and a knowledgebase of cranial surgical procedures. The operations also include receiving 5004 a user selection of one of the preset trajectories. The operations also include determining 5006 the entry point on the patient's skull based on the selected one of the preset trajectories. The operations also include generating 5008 the surgical trajectory plan based on the user designation of the target point and the determined the entry point.

In some embodiments, operations to generate 5008 the surgical trajectory plan based on the user designation of the target point and the determined the entry point include defining the target point and the entry point in the surgical trajectory plan as locations relative to the image coordinate system.

In some embodiments, operations to generate 5008 the surgical trajectory plan based on the user designation of the target point and the determined the entry point include defining the target point and the entry point in the surgical trajectory plan as location relative to the first and second radiological patient images.

In some embodiments, operations to obtain 4704 the surgical trajectory plan defining the entry point on the patient's skull and the target point in the patient's brain captured in the merged first and second radiological patient images include displaying a graphical surgical instrument representing a physical surgical instrument to be used during a cranial surgical procedure on the patient. The operations also include controlling angular orientation and location of the graphical surgical instrument displayed relative to the entry point on the patient's skull and the target point in the patient's brain captured in the merged first and second radiological patient images responsive to receipt of user inputs. The operations also include storing an indication of the angular orientation and location of the graphical surgical instrument in the surgical trajectory plan.

When selecting a preset trajectory, the surgeon plans their trajectories and locations for surgical implants on the patient images. A trajectory is defined by the entry point on the patient's skull, and the target point in the brain. The user can create and compare the trajectories on all of the co-registered MR and CT image sets that are part of the case.

Preset trajectories are available for the surgeon to choose from when creating a new trajectory. Preset trajectories, or "presets", are defined in the image coordinate system. The system's default preset coordinates are obtained from published literature. The software provides a citation for the source of each of the default presets. A pop-up screen allows selection of trajectories from previous cases, literature, or previous patients. Select the desired trajectory. Once selected, the presets can be viewed on patient images.

When selecting a new trajectory, the user can manually plan the target point and entry point on any image that has been added to the case when starting from either a preset trajectory or an empty trajectory.

To create a new trajectory, select the "+" button. The trajectory is named and its color and width are defined prior to setting the target and entry points. Click "Set Target" or "Set Entry" to define the target point and entry point, respectively. To adjust the target and entry points, click "Modify Target" or "Modify Entry," respectively. The "+" button is selected to add more trajectories.

When defining a new preset trajectory, the user can save a planned trajectory as a custom preset trajectory for use in future cases. A preset is defined by the coordinates of the target point and the entry angles within the image coordinate system. The target coordinates are entered in millimeters (mm) or as a percentage of the distance from AC to PC. Enter the sagittal and coronal entry angles.

Next, the patient is prepared by fixing the patient to the patient fixation and stabilizing the patient. Figure 37 illustrates the patient fixation and patient stabilization.

To fix the patient to the patient fixation, the patient fixation hardware is attached to the patient. Supported fixation systems include the Leksell Frame, CRW Frame, or 3-pin fixation, such as the Mayfield Skull Clamp.

To stabilize the patient, the O.R. table is adjusted to the desired height for the procedure. Power is disconnected from the table to prevent any unintended motion during the surgery. The Patient Stabilization Stand is positioned so that the distal end of the upper arm is at the superior edge of the operating table. Positioning of the stand should be optimized to avoid obstructing operating room staff and to allow access for any intraoperative imaging systems that will be used during the procedure. The corresponding fixation adapter is attached to the patient fixation hardware. The adapter is attached to the distal end of the stand. Once desired positioning of the patient and the stand are achieved, tighten all adjustment knobs on the stand and pull the Wheel Retractor Handle to lock in place.

Next, the patient position is registered by selecting the method, verifying instruments, and selecting a patient registration method. Registration is the process of registering the physical patient with the surgical plan.

In some embodiments, the operations performed by the cranial surgery planning system further include determining occurrence of a first condition when a marker tracking camera can track reflective markers that are on a cranial fluoroscopy registration fixture and determining occurrence of a second condition when the marker tracking camera can track dynamic reference base markers attached to a robot arm and/or an end-effector of a surgical robot. The operations also further include allowing operations to be performed to obtain the first radiological patient image of cranial structure of the patient along a first plane and to obtain the second radiological patient image of the cranial structure of the patient along a second plane that is angularly offset to the first plane while the fist and second conditions continue to occur.

In some embodiments, the operations performed by the cranial surgery planning system further include obtaining the first radiological patient image of cranial structure of the patient and the fiducials along a first plane and to obtain the second radiological patient image of the cranial structure of the patient and the fiducials along a second plane that is angularly offset to the first plane after a registration fixture includes fiducials is attached to cranial structure of the patient.

In some embodiments, the operations performed by the cranial surgery planning system further include registering locations of the fiducials on the registration frame to locations of the fiducials captured in the first and second radiological patient images. The operations also further include displaying a three dimensional graphical representation of the registration fixture overlaid on a three dimensional graphical representation of cranial structure captured in the first and second radiological patient images, based on the registration of the locations on the fiducials on the registration frame to the locations of the fiducials captured in the first and second radiological patient images.

In some embodiments, the registration fixture further includes reflective markers. Additionally, the operations performed by the cranial surgery planning system further include receiving locations of the reflective markers tracked by a camera tracking system relative to an optical coordinate system. The operations also further include registering locations of the fiducials on the registration frame in the image coordinate system to locations of the reflective markers in the optical coordinate system.

In some embodiments, the operations performed by the cranial surgery planning system further include obtaining the first radiological patient image of cranial structure of the patient and the registration fixture along a first plane and to obtain the second radiological patient image of the cranial structure of the patient and the registration fixture along a second plane that is angularly offset to the first plane after a registration fixture is attached to cranial structure of the patient.

There are four available methods to register the patient:
- Intraoperative CT (ICT) Registration
- CT-Fluoroscopy Registration
- Leksell Frame Registration
- CRW Frame Registration.

The desired patient registration method is selected to follow the correct workflow for that method of registration.

The instrument verification step is used for all registration methods. The instruments must be assembled prior to verification. Verify each instrument as follows:
1. Place the tip of the instrument to be verified into a verification divot in the Interchangeable Guide End Effector, CDRB, or FRA.
2. Ensure the instrument is visible and held steady.
3. A pop-up screen appears on the verify instruments page to indicate the verification progress.

Once verification is complete, verification status is indicated on the screen. If verification has failed (represented as a red crossed circle), verification must be repeated until it is successful (represented as a green circle).

Intraoperative Computed tomography (ICT) registration is discussed below.

When ICT registration is selected, the patient reference is attached to the patient fixation hardware prior to registration. If using the Leksell Frame for patient fixation, the Leksell FRA or CDRB can be used as a patient reference. If using the CRW Frame for patient fixation, the CRW FRA or CDRB can be used as a patient reference. If using 3-pin fixation, the CDRB must be used as the patient reference. When using an FRA, the FRA is attached to the frame base. When using the CDRB, the Cranial Articulating Arm is attached to the fixation adapter and then attach the CDRB to the arm. Disposable reflective markers are attached to the marker posts of the patient reference. Position the reflective markers on the CDRB in the direction of the camera. Care should be taken with initial placement of the patient reference to avoid interference with the surgical procedure.

The Surveillance Marker Holder may be attached rigidly to a patient fixation device to track the relative distance to the patient reference to monitor unwanted shifts in the patient reference during the procedure. A disposable reflective marker is attached to the marker post on the holder.

To register the fixture setup of the ICT registration fixture, the ICT Registration Clamp is placed on the registration fixture and rotate 90° to secure. The lock post is pressed from the underside and rotate the pin 90° until the pin is seated to secure the fixture.

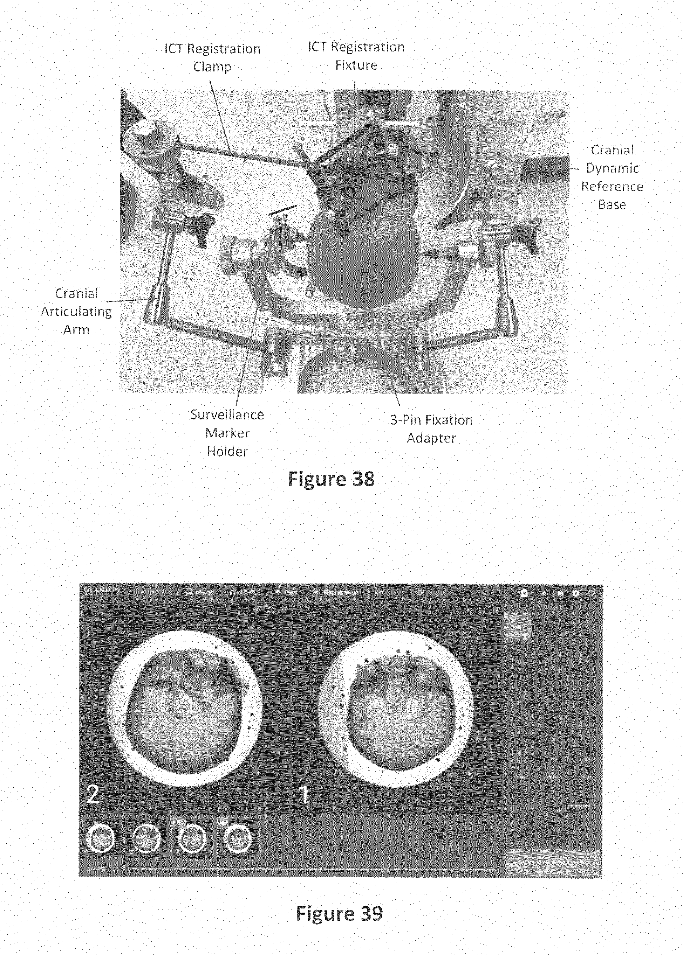

The ICT Registration Clamp is attached to the Cranial Articulating Arm. The arm is adjusted such that the metal fiducials in the registration fixture are as close to the surgical site as possible while keeping the optical markers visible to the camera, as illustrated in Figure 38. Figure 38 illustrates an example of an ICT Registration Clamp attached to the Cranial Articulating Arm. Only the metal fiducials embedded in the fixture need to be in the 3D image (not the reflective markers). It is important that the ICT Registration Fixture does not move between the image acquisition and performing an anatomical landmark check. To remove the ICT Registration Fixture, the ICT Registration Clamp is unthreaded from the Cranial Articulating Arm.

The user is prompted to confirm the setup of the surgical site for ICT image collection. This ensures that all navigated items are visible by the camera. After ICT registration setup is complete, a snapshot is taken to record the position of the patient reference and the ICT.

An intraoperative CT imaging system is used to acquire a CT image that contains patient anatomy and the ICT Registration Fixture. The image can be loaded from a USB drive or hard drive. If the image is transferred via Ethernet, it automatically appears on the hard drive when transfer is complete.

ICT fiducials are detected. The seven ICT fiducials are displayed on the right sidebar of the cranial application. Each fiducial includes a view that shows the combination of three orthogonal planes centered on the detected fiducial. An accurately detected fiducial should appear as a circle in the cranial application. Clicking on any fiducial in the right sidebar centers that fiducial in the viewports and allows the user to modify its location in any 2D view. The software displays the residual Fiducial Registration Error after optimizing the fit between the detected ICT fiducials and the nominal ICT fiducial spacing. A lower value indicates a more accurate registration.

The ICT image must be merged to the image coordinate system. This is achieved by merging the image to the master image or to any other image that is already merged to the master image.

After the ICT fiducials are confirmed, the user is prompted to transfer the registration from the ICT to the patient reference array to complete registration.

Computed tomography (CT)-Fluoroscopy registration is discussed below.

When setting up registration of CT-Fluoroscopy, the Fluoroscopy Registration Fixture is attached to the image intensifier on the C-arm. The knob on the clamp is rotated clockwise until tight. Ensure that the patient reference is visible to the camera after the C-Arm is in place.

The Surveillance Marker Holder can be attached rigidly to a patient fixation device to track the relative distance to the patient reference to monitor unwanted shifts in the patient reference during the procedure. A disposable reflective marker is attached to the marker post on the holder.

To select CT image and patient reference, the user must first select the 3D image and patient reference to be used for registration. The registration 3D image is selected in the dropdown menu on the right sidebar. All CT images that are merged to the master image coordinate system are available to be used as the registration CT image. The patient reference to be used for registration is displayed on the bottom half of the right sidebar. The selected patient reference is clicked on to cycle through all available patient references

To acquire and register the image, two fluoroscopic images (one anterior posterior (AP) and one lateral) of the patient are acquired. The lateral image can be acquired as a true lateral or at any angle at least 30° away from the AP image. The user should select the lateral image angle to maximize patient content in the image and to minimize metal artifacts from patient fixation hardware.

The following four conditions must be met prior to acquiring fluoroscopic images:
1. The patient reference is visible by the camera.
2. The Fluoroscopy Registration Fixture is attached to the C-arm and visible by the camera.
3. The connection of the cranial surgery planning system (e.g., ExcelsiusGPS system) with the C-arm is active.
4. The C-arm is stationary.

Each indicator on the right sidebar turns green when ready for image capture. When all four indicators are green, intraoperative fluoroscopic images are acquired until all desired images are captured. Figure 39 illustrates an example of image acquisition and registration in the cranial application.

As images are acquired, the user may assign the image as AP or Lateral, or the user may delete the image. Once the desired AP and Lateral images are selected, click the "Run" button in the right sidebar to run the registration algorithm.

When verifying registration, registration is visually verified prior to navigation. The Digitally Reconstructed Radiograph (DRR) created from the CT image and the fluoroscopic images are alternated between to confirm proper registration. A merge scale (1 to 10) is used to confirm image registration. Scores 6-10 indicate good image alignment. For scores 5 or less, double check the images, perform a landmark check or retake images before proceeding. Click the "Next" button to complete.

Leksell frame registration is discussed below.

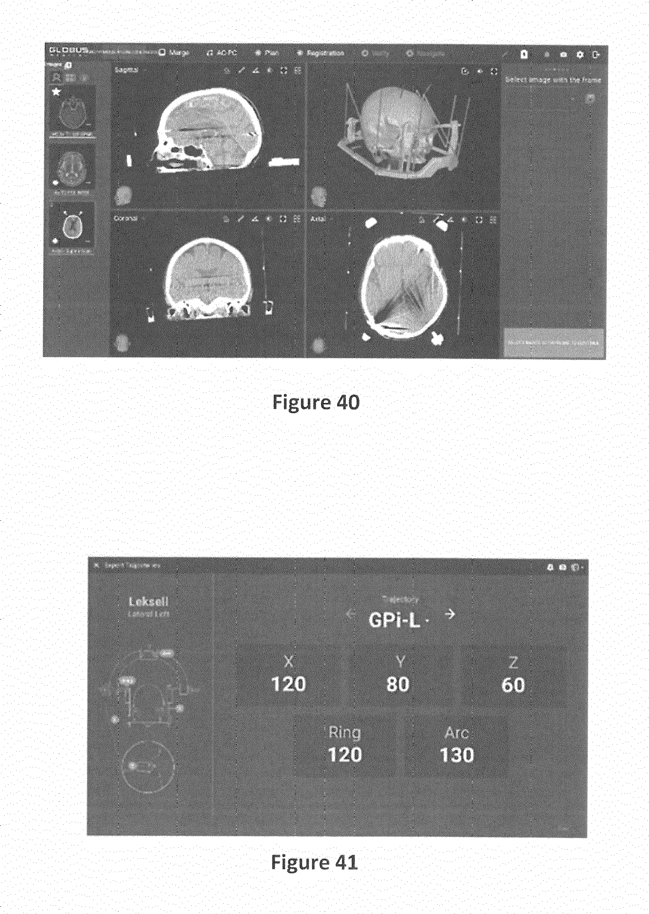

Figure 40 illustrates an example of image acquisition and registration of a Leksell frame in the cranial application.

When acquiring and importing images of the Leksell frame, the Leksell CT Localizer is attached to the Leksell frame base. a CT image of the patient is acquired with the frame and frame localizer attached. Ensure that the entirety of the frame localizer is visible in the CT image. The CT image can be acquired preoperatively or intraoperatively, provided that a rigid connection between the frame base and the patient is maintained from the time the CT image is acquired until the surgery is performed.

To perform patient reference attachment, the localizer is removed and the FRA is attached to the frame base. Disposable reflective markers are attached to the marker posts on the patient reference.

The Surveillance Marker Holder can be attached rigidly to a patient fixation device to track the relative distance to the patient reference to monitor unwanted shifts in the patient reference during the procedure. A disposable reflective marker is attached to the marker post on the holder.

When performing Leksell localizer fiducial detection, two slices of the CT image are extracted and a frame detection algorithm is performed within the software on each slice to detect the intersections with the localizer patterns. The user must verify and/or modify the detected intersection locations one at a time by clicking on the points in the list on the right sidebar. After all fiducials are placed, the frame registration is calculated.

When performing Leksell image merge, the CT image must be merged to the master image coordinate system. This is achieved by merging the image to the master image or to any other image that is already merged to the master image. The software asks the user to confirm the image merge.

The calculated frame ring and arc coordinates for each of the planned trajectories may be obtained. The coordinates can be used to align the Leksell ring and arc to the selected trajectory if the robotic arm is not being used.

Figure 41 illustrates an example of coordinates of a trajectory in the cranial application.

Cosman-Roberts-Wells (CRW) frame registration is discussed below.

When acquiring and importing images of CRWframes, the CRW localizer is first attached to the CRW frame base.

A CT image of the patient is acquired with the frame and frame localizer attached. Ensure that the entirety of the frame localizer is present in the CT image. The CT image can be acquired preoperatively or intraoperatively, provided that a rigid connection between the frame base and the patient is maintained from the time the CT image is acquired until the surgery is performed.

The localizer is removed and the FRA is attached to the frame base. Disposable reflective markers are attached to the marker posts of the patient reference.

The Surveillance Marker Holder can be attached rigidly to a patient fixation device to track the relative distance to the patient reference to monitor unwanted shifts in the patient reference during the procedure. A disposable reflective marker is attached to the marker post of the Surveillance Marker Holder.

When detecting CRW localizer fiducial, One slice of the CT image is extracted and a frame detection algorithm is performed within the software on the slice to detect the intersections with the localizer patterns. The user must verify and/or modify the detected intersection locations one at a time. After all fiducials are placed, frame registration is calculated.

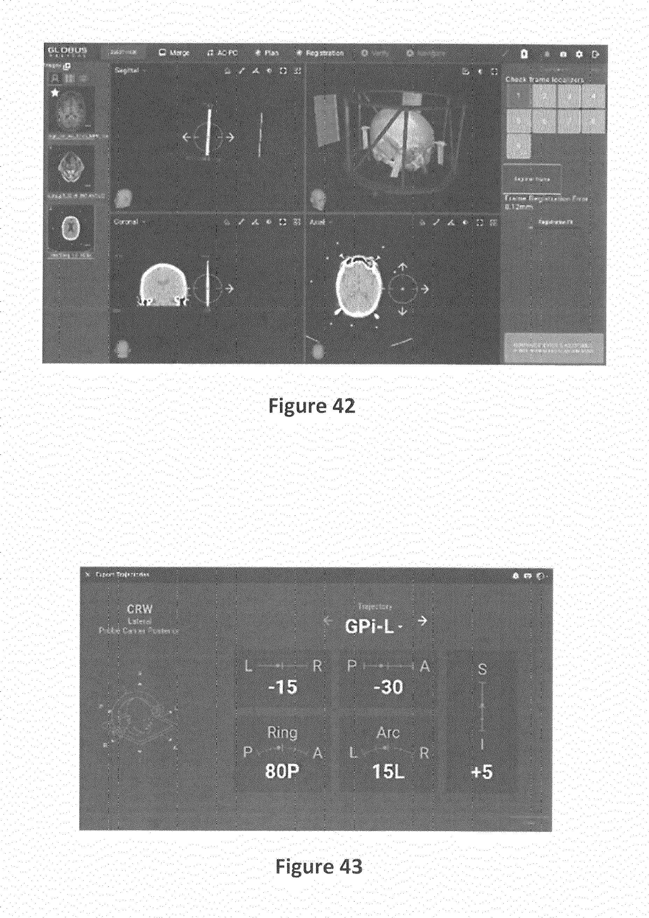

Figure 42 illustrates an example of CRW localizer fiducial detection in the cranial application.

When merging CRW images, the CT image must be merged to the master image coordinate system. This is achieved by merging the image to the master image or to any other image that is already merged to the master image. The software asks the user to confirm the image merge.

The calculated frame ring and arc coordinates are then obtained for each of the planned trajectories. The coordinates can be used to align the CRW ring and arc to the selected trajectory if the robotic arm is not being used.

Figure 43 illustrates an example of calculated frame ring and arc coordinates obtained for each of the planned trajectories in the cranial application.

Next, landmarks are checked to verify the registration.. After registration has been completed, a landmark check or verification should be performed to ensure that the registration was successfully calculated. Using the Verification Probe or Trajectory Probe, touch an anatomical landmark and verify that the corresponding location is shown on the system monitor. This process can be repeated using, e.g., 2-3 landmarks.

Register the surveillance marker by pointing the Verification Probe or Trajectory Probe at the surveillance marker without touching it. The surveillance marker box turns green when activated. The surveillance marker can be used to provide additional verification that the patient reference does not move during the procedure. If the surveillance marker indicates significant movement of the patient reference, perform an anatomical landmark check. If the landmark check is satisfactory, re-register the surveillance marker. If the landmark check fails, re-register the patient.

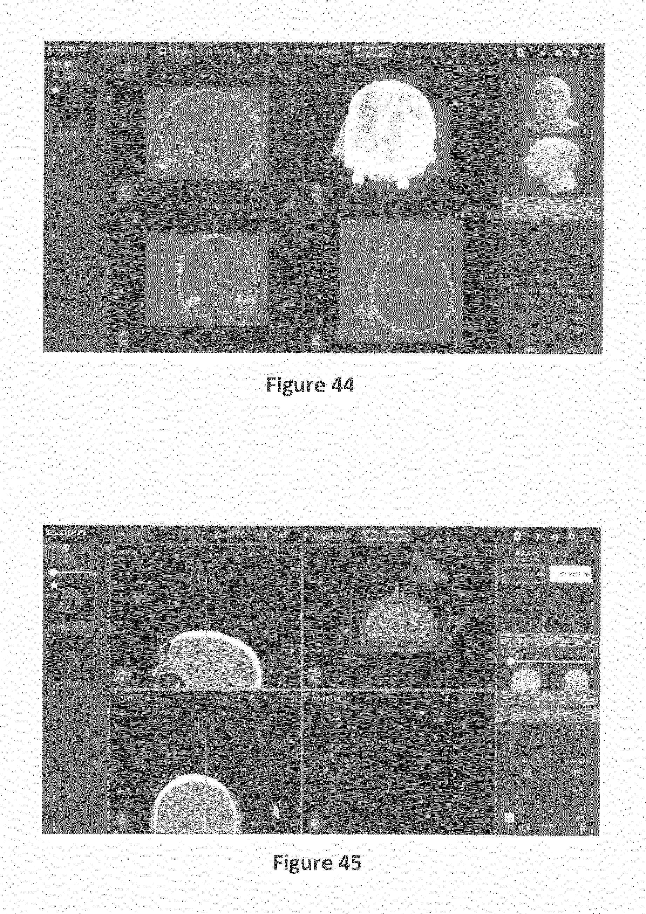

If the ICT Registration Method was used, the ICT Registration Fixture and Clamp are removed before proceeding. Figure 44 illustrates an example of images in the cranial application after the ICT Registration Fixture and Clamp are removed.

Next, the selected trajectory is navigated.

The Navigate page of the cranial application allows the user to visualize the navigated instruments and trajectory alignment with respect to patient anatomy, according to the trajectory plan. The robotic arm precisely aligns the Interchangeable Guide End Effector to the planned trajectory.

To activate a trajectory plan, select the desired trajectory on the right of the screen. A trajectory plan is active when the trajectory label is highlighted and the robotic arm can be moved by the bracelet or pressing the foot pedal. The robotic arm first moves up to clear obstacles in the surgical field, then onto the trajectory, then down along the trajectory. Once on the trajectory, the robotic arm moves up/down along the trajectory but does not move off of the trajectory unless the trajectory plan is deselected.

The instrument or implant is then inserted according to the current trajectory to the desired depth. The surveillance marker is monitored during the procedure. If the surveillance marker indicates significant movement of the patient reference, an anatomical landmark check is performed. If the landmark check is satisfactory, the surveillance marker is re-registered. If the landmark check fails, the patient is re-registered.

Figure 45 illustrates an example of images in the cranial application used to monitor the surveillance markers of instruments or implants during a procedure.

If the Leksell or CRW frame registration method was used, frame coordinates are calculated to display the Leksell or CRW frame coordinates in the cranial application.

Registration transfer from FRA to CDRB may be performed if the active patient reference is the FRA. This is helpful if the FRA needs to be removed from the frame base during the procedure.

Next, the accuracy of the selected trajectory is verified with at least one Postop Image.

Implant placement accuracy can be verified by acquiring a postoperative CT image, merging it to the master image coordinate system, and identifying the implant in the postoperative image.

After acquiring the postoperative image, import the CT image to the hard drive, and merge the image to the master image coordinate system. The trajectory is selected a virtual lead object that represents the implant is created. Up to four contact positions along each implant are defined. Once contact positions are defined, the software reports the Euclidean distance, in millimeters (mm), between each contact position and the target position, as well as the shortest distance between each contact and the planned trajectory. An overall angle, in degrees, between the planned trajectory and the detected lead is displayed.

Figure 46 illustrates an example of implant placement accuracy verification in the cranial application.

In some embodiments, the cranial surgery planning system includes a surgical robot having a robot base, a robot arm coupled to the robot base, and an end-effector coupled to the robot arm, the end-effector configured to guide movement of a surgical instrument. The cranial surgery planning system also includes a camera tracking system configured to output tracking information indicating locations of reflective markers on the surgical robot and locations of reflective markers on a registration fixture attached to cranial structure of the patient. The at least one processor performs operations to obtain the first and second patient images for the patient and to track pose of the end-effector relative to cranial structure captured in the first and second patient images based on the tracking information from the camera tracking system.

In some embodiments, the at least one processor performs operations to receive the surgical trajectory plan and to control movement of at least one motor operatively connected to move the robot arm relative to the robot base, based on the surgical trajectory plan.

In some embodiments, the operations performed by the cranial surgery planning system further include determining a target pose for the end-effector based on the surgical trajectory plan. The operations also further include generating steering information based on the target pose for the surgical trajectory plan and a present tracked pose of the end-effector indicated by the tracking information, the steering information indicating where the end-effector needs to be moved relative to the cranial structure of the patient.

In some embodiments, the operations performed by the cranial surgery planning system further include controlling movement of the at least one motor based on the steering information to guide movement of the end-effector so the end-effector becomes positioned with the target pose relative to the cranial structure of the patient.
Further Definitions and Embodiments:
In the above-description of various embodiments of the present disclosure, aspects of the present disclosure may be illustrated and described herein in any of a number of patentable classes or contexts including any new and useful process, machine, manufacture, or composition of matter, or any new and useful improvement thereof. Accordingly, aspects of the present disclosure may be implemented in entirely hardware, entirely software (including firmware, resident software, micro-code, etc.) or combining software and hardware implementation that may all generally be referred to herein as a "circuit," "module," "component," or "system." Furthermore, aspects of the present disclosure may take the form of a computer program product comprising one or more computer readable media having computer readable program code embodied thereon.

Any combination of one or more computer readable media may be used. The computer readable media may be a computer readable signal medium or a computer readable storage medium. A computer readable storage medium may be, for example, but not limited to, an electronic, magnetic, optical, electromagnetic, or semiconductor system, apparatus, or device, or any suitable combination of the foregoing. More specific examples (a non-exhaustive list) of the computer readable storage medium would include the following: a portable computer diskette, a hard disk, a random access memory (RAM), a read-only memory (ROM), an erasable programmable read-only memory (EPROM or Flash memory), an appropriate optical fiber with a repeater, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device. Program code embodied on a computer readable signal medium may be transmitted using any appropriate medium, including but not limited to wireless, wireline, optical fiber cable, RF, etc., or any suitable combination of the foregoing.

Computer program code for carrying out operations for aspects of the present disclosure may be written in any combination of one or more programming languages, including an object oriented programming language such as Java, Scala, Smalltalk, Eiffel, JADE, Emerald, C++, C#, VB.NET, Python or the like, conventional procedural programming languages, such as the "C" programming language, Visual Basic, Fortran 2003, Perl, COBOL 2002, PHP, ABAP, dynamic programming languages such as Python, Ruby and Groovy, or other programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider) or in a cloud computing environment or offered as a service such as a Software as a Service (SaaS).

Aspects of the present disclosure are described herein with reference to flowchart illustrations and/or block diagrams of methods, apparatus (systems), and computer program products according to embodiments of the disclosure. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable instruction execution apparatus, create a mechanism for implementing the functions/acts specified in the flowchart and/or block diagram block or blocks.

These computer program instructions may also be stored in a computer readable medium that when executed can direct a computer, other programmable data processing apparatus, or other devices to function in a particular manner, such that the instructions when stored in the computer readable medium produce an article of manufacture including instructions which when executed, cause a computer to implement the function/act specified in the flowchart and/or block diagram block or blocks. The computer program instructions may also be loaded onto a computer, other programmable instruction execution apparatus, or other devices to cause a series of operational steps to be performed on the computer, other programmable apparatuses or other devices to produce a computer implemented process such that the instructions which execute on the computer or other programmable apparatus provide processes for implementing the functions/acts specified in the flowchart and/or block diagram block or blocks.

It is to be understood that the terminology used herein is for the purpose of describing particular embodiments only and is not intended to be limiting of the invention. Unless otherwise defined, all terms (including technical and scientific terms) used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of this specification and the relevant art and will not be interpreted in an idealized or overly formal sense unless expressly so defined herein.

The flowchart and block diagrams in the figures illustrate the architecture, functionality, and operation of possible implementations of systems, methods, and computer program products according to various aspects of the present disclosure. In this regard, each block in the flowchart or block diagrams may represent a module, segment, or portion of code, which comprises one or more executable instructions for implementing the specified logical function(s). It should also be noted that, in some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts, or combinations of special purpose hardware and computer instructions.

The terminology used herein is for the purpose of describing particular aspects only and is not intended to be limiting of the disclosure. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items. Like reference numbers signify like elements throughout the description of the figures.

The corresponding structures, materials, acts, and equivalents of any means or step plus function elements in the claims below are intended to include any disclosed structure, material, or act for performing the function in combination with other claimed elements as specifically claimed. The description of the present disclosure has been presented for purposes of illustration and description, but is not intended to be exhaustive or limited to the disclosure in the form disclosed. Many modifications and variations will be apparent to those of ordinary skill in the art without departing from the scope and spirit of the disclosure. The aspects of the disclosure herein were chosen and described in order to best explain the principles of the disclosure and the practical application, and to enable others of ordinary skill in the art to understand the disclosure with various modifications as are suited to the particular use contemplated.

The invention could be inter alia defined by the following examples:
1. A cranial surgery planning system comprising: at least one network interface connectable to obtain radiological patient images generated by a radiological image scanner; a display device; at least one processor; and at least one memory storing program code that is executed by the at least one processor to perform operations to obtain through the at least one network interface a first radiological patient image of cranial structure of a patient along a first plane and obtain a second radiological patient image of the cranial structure of the patient along a second plane that is angularly offset to the first plane, merge the first and second radiological patient images to an image coordinate system, and obtain a surgical trajectory plan defining an entry point on the patient's skull and a target point in the patient's brain captured in the merged first and second radiological patient images.
2. The cranial surgery planning system of Example 1, wherein the first and second radiological patient images are angularly offset in a range between 90° and 30°.
3. The cranial surgery planning system of Example 1, wherein to merge the first and second radiological patient images to the image coordinate system, the at least one processor performs operations to: generate a three dimensional graphical representation in the image coordinate system of cranial structure captured in the first and second radiological patient images.
4. The cranial surgery planning system of Example 1, wherein to obtain the surgical trajectory plan defining the entry point on the patient's skull and the target point in the patient's brain captured in the merged first and second radiological patient images, the at least one processor performs operations to: receive a user designation of the entry point on the patient's skull and the target point in the patient's brain defined relative to the image coordinate system; and store the user designation of the entry point and the target point in the surgical trajectory plan.
5. The cranial surgery planning system of Example 1, wherein to obtain the surgical trajectory plan defining the entry point on the patient's skull and the target point in the patient's brain captured in the merged first and second radiological patient images, the at least one processor performs operations to: receive a user designation of the target point in the patient's brain defined relative to the image coordinate system; generate a set of preset trajectories based on the target point and a knowledgebase of cranial surgical procedures; receive a user selection of one of the preset trajectories; determining the entry point on the patient's skull based on the selected one of the preset trajectories; and generate the surgical trajectory plan based on the user designation of the target point and the determined the entry point.
6. The cranial surgery planning system of Example 5, wherein the at least one processor performs operations to: generate a set of preset trajectories based on the target point and a knowledgebase of cranial surgical procedures, by displaying graphical representations of the trajectories as overlays on the first and second radiological patient images; and receive the user selection of one of the preset trajectories, by receiving the user selection of one of the displayed graphical representations of the trajectories.
7. The cranial surgery planning system of Example 5, wherein to generate the surgical trajectory plan based on the user designation of the target point and the determined the entry point, the at least one processor performs operations to: define the target point and the entry point in the surgical trajectory plan as locations relative to the image coordinate system.
8. The cranial surgery planning system of Example 5, wherein to generate the surgical trajectory plan based on the user designation of the target point and the determined the entry point, the at least one processor performs operations to: define the target point and the entry point in the surgical trajectory plan as location relative to the first and second radiological patient images.
9. The cranial surgery planning system of Example 1, wherein to obtain the surgical trajectory plan defining the entry point on the patient's skull and the target point in the patient's brain captured in the merged first and second radiological patient images, the at least one processor performs operations to: display a graphical surgical instrument representing a physical surgical instrument to be used during a cranial surgical procedure on the patient; responsive to receipt of user inputs, control angular orientation and location of the graphical surgical instrument displayed relative to the entry point on the patient's skull and the target point in the patient's brain captured in the merged first and second radiological patient images; and store an indication of the angular orientation and location of the graphical surgical instrument in the surgical trajectory plan.
10. The cranial surgery planning system of Example 1, wherein the at least one processor performs operations to: determine occurrence of a first condition when a marker tracking camera can track reflective markers that are on a cranial fluoroscopy registration fixture, and determine occurrence of a second condition when the marker tracking camera can track dynamic reference base markers attached to a robot arm and/or an end-effector of a surgical robot; and while both the first and second conditions continue to occur, allow operations to be performed to obtain the first radiological patient image of cranial structure of the patient along a first plane and to obtain the second radiological patient image of the cranial structure of the patient along a second plane that is angularly offset to the first plane.
11. The cranial surgery planning system of Example 1, wherein the at least one processor performs operations to: after a registration fixture includes fiducials is attached to cranial structure of the patient, obtain the first radiological patient image of cranial structure of the patient and the fiducials along a first plane and to obtain the second radiological patient image of the cranial structure of the patient and the fiducials along a second plane that is angularly offset to the first plane.
12. The cranial surgery planning system of Example 11, wherein the at least one processor performs operations to: register locations of the fiducials on the registration frame to locations of the fiducials captured in the first and second radiological patient images; and display a three dimensional graphical representation of the registration fixture overlaid on a three dimensional graphical representation of cranial structure captured in the first and second radiological patient images, based on the registration of the locations on the fiducials on the registration frame to the locations of the fiducials captured in the first and second radiological patient images.
13. The cranial surgery planning system of Example 12, wherein: the registration fixture further includes reflective markers; and the at least one processor further performs operations to: receive locations of the reflective markers tracked by a camera tracking system relative to an optical coordinate system; and register locations of the fiducials on the registration frame in the image coordinate system to locations of the reflective markers in the optical coordinate system.
14. The cranial surgery planning system of Example 1, wherein the at least one processor performs operations to: after a registration fixture is attached to cranial structure of the patient, obtain the first radiological patient image of cranial structure of the patient and the registration fixture along a first plane and to obtain the second radiological patient image of the cranial structure of the patient and the registration fixture along a second plane that is angularly offset to the first plane.
15. The cranial surgery planning system of Example 1, further comprising: a surgical robot having a robot base, a robot arm coupled to the robot base, and an end-effector coupled to the robot arm, the end-effector configured to guide movement of a surgical instrument; and camera tracking system configured to output tracking information indicating locations of reflective markers on the surgical robot and locations of reflective markers on a registration fixture attached to cranial structure of the patient, wherein the at least one processor performs operations to obtain the first and second patient images for the patient and to track pose of the end-effector relative to cranial structure captured in the first and second patient images based on the tracking information from the camera tracking system.
16. The cranial surgery planning system of Example 15, wherein the at least one processor performs operations to receive the surgical trajectory plan and to control movement of at least one motor operatively connected to move the robot arm relative to the robot base, based on the surgical trajectory plan.
17. The cranial surgery planning system of Example 16, wherein the at least one processor performs operations to: determine a target pose for the end-effector based on the surgical trajectory plan; and generate steering information based on the target pose for the surgical trajectory plan and a present tracked pose of the end-effector indicated by the tracking information, the steering information indicating where the end-effector needs to be moved relative to the cranial structure of the patient.
18. The cranial surgery planning system of Example 17, wherein the at least one processor performs operations to: control movement of the at least one motor based on the steering information to guide movement of the end-effector so the end-effector becomes positioned with the target pose relative to the cranial structure of the patient.
19. A method of operating a cranial surgery planning system, the method comprising: obtaining a first radiological patient image of cranial structure of a patient along a first plane and obtain a second radiological patient image of the cranial structure of the patient along a second plane that is angularly offset to the first plane; merging the first and second radiological patient images to an image coordinate system; and obtaining a surgical trajectory plan defining an entry point on the patient's skull and a target point in the patient's brain captured in the merged first and second radiological patient images.
20. A computer program product for operating a cranial surgery planning system, the computer program product comprising: a non-transitory computer readable medium storing instructions executable by at least one processor to perform operations to obtain a first radiological patient image of cranial structure of a patient along a first plane and obtain a second radiological patient image of the cranial structure of the patient along a second plane that is angularly offset to the first plane, merge the first and second radiological patient images to an image coordinate system, and obtain a surgical trajectory plan defining an entry point on the patient's skull and a target point in the patient's brain captured in the merged first and second radiological patient images.

## Claims

1. A cranial surgery planning system comprising:
at least one network interface configured to obtain radiological patient images generated by a radiological image scanner;
a display device;
at least one processor; and
at least one memory storing program code that is executed by the at least one processor to perform operations to
obtain through the at least one network interface a first radiological patient image of cranial structure of a patient along a first plane and obtain a second radiological patient image of the cranial structure of the patient along a second plane that is angularly offset to the first plane,
merge the first and second radiological patient images to an image coordinate system, and obtain a surgical trajectory plan defining an entry point on the patient's skull and a target point in the patient's brain captured in the merged first and second radiological patient images.

2. The cranial surgery planning system of Claim 1, wherein the first and second radiological patient images are angularly offset in a range between 90° and 30°.

3. The cranial surgery planning system of Claim 1 or 2, wherein to merge the first and second radiological patient images to the image coordinate system, the at least one processor performs operations to generate a three dimensional graphical representation in the image coordinate system of cranial structure captured in the first and second radiological patient images.

4. The cranial surgery planning system of any one of Claims 1-3, wherein to obtain the surgical trajectory plan defining the entry point on the patient's skull and the target point in the patient's brain captured in the merged first and second radiological patient images, the at least one processor performs operations to receive a user designation of the entry point on the patient's skull and the target point in the patient's brain defined relative to the image coordinate system; and store the user designation of the entry point and the target point in the surgical trajectory plan.

5. The cranial surgery planning system of any one of Claims 1-4, wherein to obtain the surgical trajectory plan defining the entry point on the patient's skull and the target point in the patient's brain captured in the merged first and second radiological patient images, the at least one processor performs operations to:
receive a user designation of the target point in the patient's brain defined relative to the image coordinate system;
generate a set of preset trajectories based on the target point and a knowledgebase of cranial surgical procedures;
receive a user selection of one of the preset trajectories;
determining the entry point on the patient's skull based on the selected one of the preset trajectories; and
generate the surgical trajectory plan based on the user designation of the target point and
the determined the entry point.

6. The cranial surgery planning system any one of Claims 1-5, wherein the at least one processor performs operations to:
generate a set of preset trajectories based on the target point and a knowledgebase of cranial surgical procedures, by displaying graphical representations of the trajectories as overlays on the first and second radiological patient images; and
receive the user selection of one of the preset trajectories, by receiving the user selection of one of the displayed graphical representations of the trajectories.

7. The cranial surgery planning system of any one of Claims 1-6, wherein to generate the surgical trajectory plan based on the user designation of the target point and the determined the entry point, the at least one processor performs operations to:
define the target point and the entry point in the surgical trajectory plan as locations relative to the image coordinate system.

8. The cranial surgery planning system of any one of Claims 5-7, wherein to generate the surgical trajectory plan based on the user designation of the target point and the determined the entry point, the at least one processor performs operations to:
define the target point and the entry point in the surgical trajectory plan as location relative to the first and second radiological patient images.

9. The cranial surgery planning system of any one of Claims 1-8, wherein to obtain the surgical trajectory plan defining the entry point on the patient's skull and the target point in the patient's brain captured in the merged first and second radiological patient images, the at least one processor performs operations to:
display a graphical surgical instrument representing a physical surgical instrument to be used during a cranial surgical procedure on the patient;
responsive to receipt of user inputs, control angular orientation and location of the graphical surgical instrument displayed relative to the entry point on the patient's skull and the target point in the patient's brain captured in the merged first and second radiological patient images; and
store an indication of the angular orientation and location of the graphical surgical instrument in the surgical trajectory plan.

10. The cranial surgery planning system any one of Claims 1-9, wherein the at least one processor performs operations to:
determine occurrence of a first condition when a marker tracking camera can track reflective markers that are on a cranial fluoroscopy registration fixture, and determine occurrence of a second condition when the marker tracking camera can track dynamic reference base markers attached to a robot arm and/or an end-effector of a surgical robot; and
while both the first and second conditions continue to occur, allow operations to be performed to obtain the first radiological patient image of cranial structure of the patient along a first plane and to obtain the second radiological patient image of the cranial structure of the patient along a second plane that is angularly offset to the first plane.

11. The cranial surgery planning system any one of Claims 1-10, wherein the at least one processor performs operations to:
after a registration fixture includes fiducials is attached to cranial structure of the patient, obtain the first radiological patient image of cranial structure of the patient and the fiducials along a first plane and to obtain the second radiological patient image of the cranial structure of the patient and the fiducials along a second plane that is angularly offset to the first plane.

12. The cranial surgery planning system of Claim 11, wherein the at least one processor performs operations to:
register locations of the fiducials on the registration frame to locations of the fiducials captured in the first and second radiological patient images; and
display a three dimensional graphical representation of the registration fixture overlaid on a three dimensional graphical representation of cranial structure captured in the first and second radiological patient images, based on the registration of the locations on the fiducials on the registration frame to the locations of the fiducials captured in the first and second radiological patient images.

13. The cranial surgery planning system of Claim 12, wherein:
the registration fixture further includes reflective markers; and
the at least one processor further performs operations to:
receive locations of the reflective markers tracked by a camera tracking system relative to an optical coordinate system; and
register locations of the fiducials on the registration frame in the image coordinate system to locations of the reflective markers in the optical coordinate system.

14. The cranial surgery planning system of any one of Claims 1-13, wherein the at least one processor performs operations to:
after a registration fixture is attached to cranial structure of the patient, obtain the first radiological patient image of cranial structure of the patient and the registration fixture along a first plane and to obtain the second radiological patient image of the cranial structure of the patient and the registration fixture along a second plane that is angularly offset to the first plane.

15. The cranial surgery planning system of any one of Claims 1-14, further comprising:
a surgical robot having a robot base, a robot arm coupled to the robot base, and an end-effector coupled to the robot arm, the end-effector configured to guide movement of a surgical instrument; and
camera tracking system configured to output tracking information indicating locations of reflective markers on the surgical robot and locations of reflective markers on a registration fixture attached to cranial structure of the patient,
wherein the at least one processor performs operations to obtain the first and second patient images for the patient and to track pose of the end-effector relative to cranial structure captured in the first and second patient images based on the tracking information from the camera tracking system.

16. A method of operating a cranial surgery planning system, the method comprising:
obtaining a first radiological patient image of cranial structure of a patient along a first plane and obtain a second radiological patient image of the cranial structure of the patient along a second plane that is angularly offset to the first plane;
merging the first and second radiological patient images to an image coordinate system; and
obtaining a surgical trajectory plan defining an entry point on the patient's skull and a target point in the patient's brain captured in the merged first and second radiological patient images.

17. A computer program product for operating a cranial surgery planning system, the computer program product comprising:
a non-transitory computer readable medium storing instructions executable by at least one processor to perform operations to
obtain a first radiological patient image of cranial structure of a patient along a first plane and obtain a second radiological patient image of the cranial structure of the patient along a second plane that is angularly offset to the first plane,
merge the first and second radiological patient images to an image coordinate system, and obtain a surgical trajectory plan defining an entry point on the patient's skull and a target point in the patient's brain captured in the merged first and second radiological patient images.
